# FASCICULE DE BREVET EUROPEEN

(11) **EP 0 708 099 B1**
(45) Date de publication et mention de la délivrance du brevet: **28.11.2001**
(21) Numéro de dépôt: 95402331.3
(22) Date de dépôt: 19.10.1995
(51) Int. Cl.: C07D 307/92, C07D 491/04, C07C 233/18, C07C 275/22, C07D 311/92, C07D 495/04, C07D 493/04, A61K 31/34

(54) **Nouveaux composés amides tricycliques, leur procédé de préparation et les compositions pharmaceutiques qui les contiennent**
Tricyclische Amidverbindungen, Verfahren zu deren Herstellung und sie enthaltende pharmazeutische Zusammensetzungen
Tricyclic amide compounds, process for their preparation and pharmaceutical compositions containing them

(30) Priorité: 21.10.1994 FR 9412581
(43) Date de publication de la demande: 24.04.1996
(73) Titulaire: LES LABORATOIRES SERVIER, 92200 Neuilly sur Seine (FR)
(72) Inventeur: Lesieur, Daniel, F-59147 Gondecourt (FR); Depreux, Patrick, F-59280 Armentieres (FR); LeClerk, Véronique, F-51000 Lille (FR); Ait Mansour, Hamid, F-59100 Roubaix (FR); Delagrange, Philippe, F-92130 Issy-les-Moulineaux (FR); Renard, Pierre, F-78000 Versailles (FR)

(56) Documents cités:
- EP-A- 0 286 515
- EP-A- 0 286 516
- EP-A- 0 447 285
- EP-A- 0 530 087
- EP-A- 0 562 956

## Description

L'invention concerne de nouveaux composés amides tricycliques, leurs procédés de préparation et les compositions pharmaceutiques qui les contiennent.

L'art antérieur décrit des structures 2,3,5,6,7,8-hexahydronaphto[2,3-b]furaniques et 5,6,7,8-tétrahydronaphto[2,3-b]furaniques utiles dans le traitement de maladies nécessitant des médications antidépressives antiagressives ou des modulateurs dopaminergiques (EP 0 286 516, EP 0 286 515).

Par ailleurs, on connaît dans la littérature des composés possédant une grande affinité pour les récepteurs mélatoninergiques, comme par exemple ceux décrits dans les demandes de brevets EP 0 562 956 et EP 0 530 087.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement de la maladie de Parkinson (J. Neurosurg 1985, 63, pp 321-341) et d'Alzheimer (Brain Research, 1990, 528, pp 170-174) De même, ces composés ont montré une activité sur certains cancers (Melatonin - clinical Perspectives, Oxford University Press, 1988, page 164-165), sur l'ovulation (Science 1987, 227, pp 714-720), et sur le diabète (Clinical endocrinology, 1986, 24, pp 359-364).

Des composés permettant d'agir sur le système mélatoninergique sont donc pour le clinicien d'excellents médicaments pour le traitement des pathologies mentionnées précédemment.

La demanderesse a découvert de nouveaux composés amides tricycliques, de structure originale, montrant une très haute affinité pour les récepteurs mélatoninergiques et présentant, in vitro et in vivo, un grand intérêt pharmacologique et thérapeutique.

L'invention concerne plus particulièrement les composés de formule (I) : 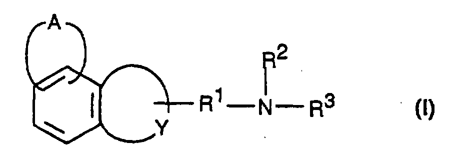 dans laquelle :
- R¹ représente une chaîne (C₁-C₄) alkylène non substituée ou substituée par un radical choisi parmi alkyle, hydroxy, alkoxycarbonyle et carboxyle ;
- R² représente un atome d'hydrogène ou un alkyle ;
- R³ représente :
   . soit un groupement de formule R³¹ 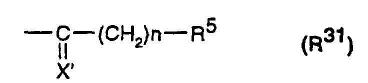 dans lequel n représente zéro ou un nombre entier de 1 à 3 et R⁵ représente un atome d'hydrogène, un alkyle non substitué ou substitué, un alcényle non substitué ou substitué, un alcynyle non substitué ou substitué, un cycloalkyle non substitué ou substitué, un dicycloalkylalkyle non substitué ou substitué ; et X' représente un atome d'oxygène ou de soufre ;
   . soit un groupement de formule R³² : 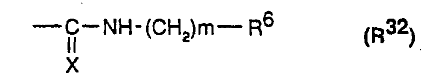 dans lequel X représente un atome d'oxygène ou de soufre,
      m représente zéro ou un nombre entier de 1 à 3
      et R⁶ représente un radical choisi parmi les même valeurs que R⁵;
- A représente une chaine de formule -O-A¹- dans laquelle A¹ est une chaine choisie parmi (C₂-C₅) alkylène, (C₂-C₅) alcénylène et (C₂-C₅) alcynylène ; A¹ étant non substitué ou substitué par un ou plusieurs groupements choisis parmi alkyle, alkoxy hydroxy et oxo,
Y formant avec le noyau benzo auquel il est lié un groupement Y¹ choisi parmi naphtalène, naphtalène partiellement hydrogène, benzofuranne, benzofuranne partiellement hydrogéné, benzothiophène, benzothiophène partiellement hydrogéné et indole ;
étant entendu que :
- l'expression "substitué" affectant les termes "alkyle", "alcényle", et "alcynyle" signifie que ces groupements sont substitués par un ou plusieurs radicaux choisis parmi halogène, alkyle et alkoxy,
- l'expression "substitué" affectant le terme "cycloalkyle" ou "dicycloalkylalkyle" signifie que ces groupements sont substitués par un ou plusieurs radicaux choisis parmi : alkyle, alkoxy, hydroxy et le groupement oxo,
- les termes "alkyle" et "alkoxy" désignent des radicaux comportant de 1 à 6 atomes de carbone,
- les termes "alcényle" et "alcynyle" désignent des radicaux insaturés de 2 à 6 atomes de carbone,
- le terme "cycloalkyle" désigne un groupement de 3 à 8 atomes de carbone, saturé ou insaturé,
leurs énantiomères et diastéréoisomères,
et leurs sels d'addition à une base pharmaceutiquement acceptable.

Particulièrement, l'invention concerne :
- les composés de formule (I) dans laquelle R¹ représente une chaîne éthylène,
- les composés de formule (I) dans laquelle R² représente un atome d'hydrogène,
- les composés de formule (I) dans laquelle R³ représente un groupement de formule R³¹,
- les composés de formule (I) dans laquelle R⁵ représente un alkyle,
- les composés de formule (I) dans laquelle R⁵ représente un groupement cycloalkyle,
- les composés de formule (I) dans laquelle R³ représente un groupement R³²,
- les composés de formule (I) dans laquelle R⁶ représente un alkyle,
- les composés de formule (I) dans laquelle R⁶ représente un cycloalkyle,
- les composés de formule (I) dans laquelle X est un atome d'oxygène,
- les composés de formule (I) dans laquelle X est un atome de soufre,
- les composés de formule (I) dans laquelle X' est un atome d'oxygène,
- les composés de formule (I) dans laquelle X' est un atome de soufre,
- les composés de formule (I) dans laquelle A¹ est une chaîne éthylène,
- les composés de formule (I) dans laquelle A¹ est une chaîne triméthylène,
- les composés de formule (I) dans laquelle A¹ est une chaîne tétraméthylène,
- les composés de formule (I) dans laquelle A¹ est une chaîne vinylène,
- les composés de formule (I) dans laquelle A¹ est une chaîne propenylène,
- les composés de formule (I) dans laquelle Y forme avec le noyau benzo auquel il est lié, un groupement naphtalène,
- les composés de formule (I) dans laquelle Y forme avec le noyau benzo auquel il est lié, un groupement tétrahydronaphtalène,
- les composés de formule (I) dans laquelle Y forme avec le noyau benzo auquel il est lié, un groupement indole.

Plus particulièrement, l'invention concerne :
- les composés de formule (I₁) 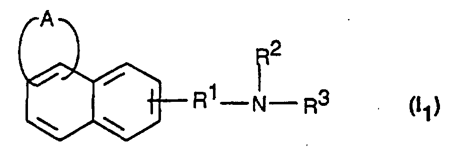
dans laquelle A, R¹, R² et R³ sont tels que définis dans la formule (I)
et les composés de formule (I₂) 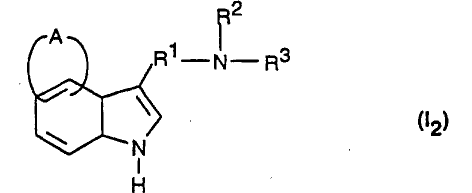 dans laquelle A, R¹, R² et R³ sont tels que définis dans la formule (I).

Par exemple, l'invention concerne les composés de formule (I₃): 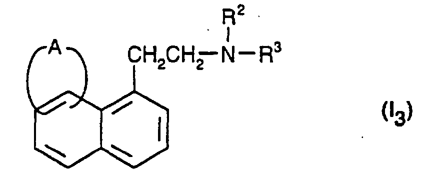 dans laquelle A, R² et R³ sont tels que définis dans la formule (I)
et les composés de formule (I₄) 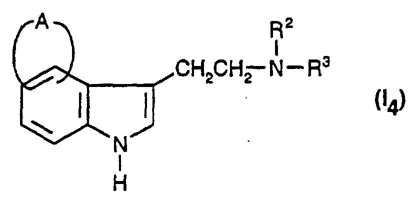 dans laquelle A, R² et R³ sont tels que définis dans la formule (I).

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle, ou hexyle.

Les radicaux alkoxy présents dans la formule (I) peuvent être choisis parmi méthoxy, éthoxy, n-propoxy, isopropoxy, n-butoxy, isobutoxy, sec-butoxy, tert-butoxy, pentyloxy, et hexyloxy.

Les halogènes présents dans la formule (I) peuvent être choisis parmi le brome, le chlore, le fluor, et l'iode.

Les cycloalkyles présents dans la formule (I) peuvent être choisis parmi cyclopropyle, cyclobutyle, cyclopentyle, cyclohexyle, cycloheptyle et cyclooctyle.

Les groupements alkylènes présents dans la formule (I) peuvent étre choisis parmi éthylène, triméthylène, tétraméthylène et pentaméthylène.

L'invention concerne également le procédé de préparation des composés de formule (I) caractérisé en ce qu'on cyclise un composé de formule (II) : 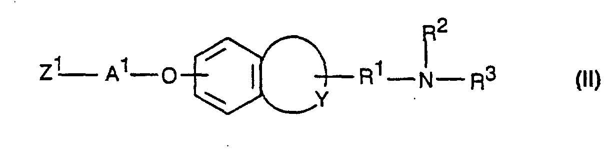 dans laquelle R¹, R², R³, A¹ et Y ont la même définition que dans la formule (I) et Z¹ représente une fonction réactive,
afin d'obtenir le composé de formule (I) correspondant, 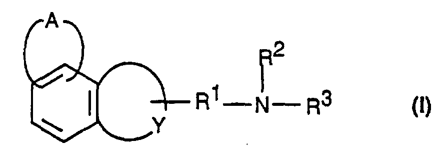 dans laquelle R¹, R², R³ et Y sont tels que définis précédemment et A est tel que défini dans la formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

L'invention concerne également un procédé de préparation des composés de formule (I) caractérisé en ce qu'on fait réagir un composé de formule (III): 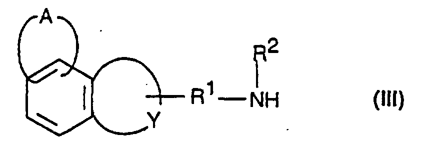 dans laquelle A, R¹, R² et Y sont telles que définis dans la formule (I),
a) avec un chlorure d'acyle de formule (IV) : 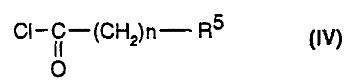 dans laquelle n et R⁵ sont tels que définis dans la formule (I), ou avec l'anhydride (symétrique ou mixte) d'acide correspondant, ou bien avec de l'acide formique,
b) ou bien avec un isocyanate de formule (V) :

   X=C=N-(CH₂)m―R⁶ **(V)**

   avec X, m et R⁶ tels que définis dans la formule (I)
afin d'obtenir, respectivement :
a) le composé de formule (I/b1) : 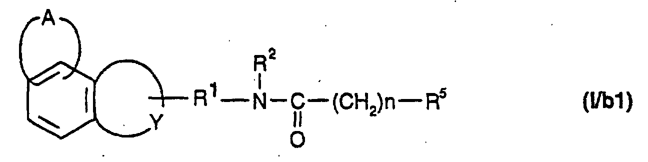 dans laquelle A, Y, R¹, R², R⁵ et n sont tels que définis précédemment,
ou b) le composé de formule (I/b2) : 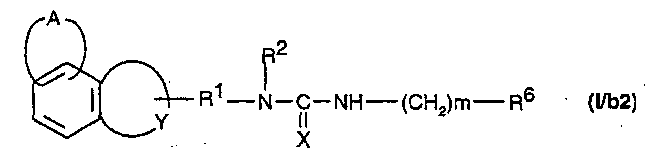 dans laquelle A, Y, R¹, R², R⁶, X et m sont tels que définis précédemment,
les composés de formule (I/b1) et (I/b2) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

Le composé de formule (I) dans lequel R³¹ représente un groupement -CS-(CH₂)ₙ-R⁵ peut également être obtenu à partir du composé de formule (I) correspondant, dans lequel R³¹ représente un groupement -CO-(CH₂)ₙ-R₅, qui est soumis à un réactif de thionation, par exemple le réactif de Lawesson.

L'invention concerne également la préparation de composés de formule (I/c1) : 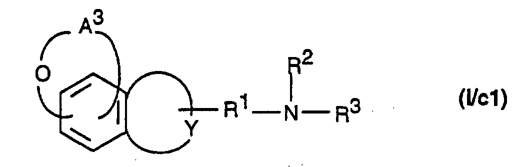 dans laquelle R¹, R², R³ et Y sont tels que définis dans la formule (I) et A³ représente une chaine (C₂-C₅) alkylène substituée par un radical hydroxy ou une chaîne (C₂-C₅) alcénylène,
caractérisé en ce que on réalise la réduction ménagée d'un composé de formule (I/c0) : 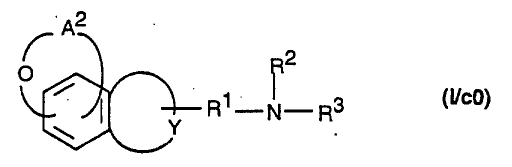 dans laquelle R¹, R², R³ et Y sont tels que définis précédemment et A² représente une chaine (C₂-C₅) alkylène substituée par un groupement oxo,
les composés de formule (I/c1) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

L'invention concerne également un procédé de préparation des composés de formule (I/d) cas particulier des composés de formule (I): 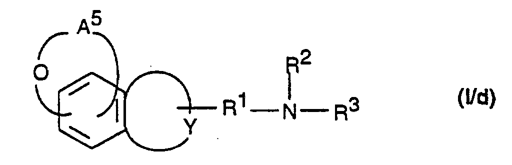 dans laquelle Y, R¹, R² et R³ sont tels que définis dans la formule (I) et A⁵ représente une chaîne (C₂-C₅) alkylène non substituée ou substituée par un radical (C₁-C₆) alkyle caractérisé en ce que un composé de formule (VI) : 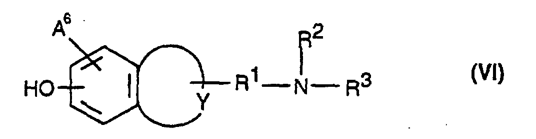 dans laquelle Y, R¹, R² et R³ sont tels que définis précédemment et A⁶ représente un radical (C₂-C₅) alcényle non substitué ou substitué par un radical (C₁-C₆) alkyle est soumis à une réaction de cyclisation,
les composés de formule (I/d) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

L'invention concerne également les composés de formule (VI) : 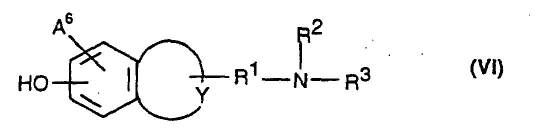 dans laquelle R¹, R², R³ et Y sont tels que définis dans la formule (I) et A⁶ représente un radical (C₂-C₅) alcényle non substitué ou substitué par un radical (C₁-C₆) alkyle, utiles comme intermédiaires de synthèse.

Les composés de formule (II) tels que décrits précédemment sont accessibles à l'homme du métier par réaction d'un composé de formule (II/a): 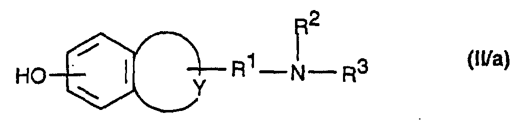 dans laquelle R¹, R², R³ et Y sont tels que définis dans la formule (I), avec un composé de formule (II/b) :

Z²-A¹-Z³ **(II/b)**

dans laquelle A¹ a la même définition que dans la formule (I), Z² représente une fonction réactive éventuellement protégée et Z³ représente un groupement partant, par exemple un atome d'halogène ou un groupement tosyle.
Par exemple Z² représente une fonction hydroxyle, carboxyle, une liaison double ou une liaison triple.

L'invention s'étend également aux composés de formule (II) : 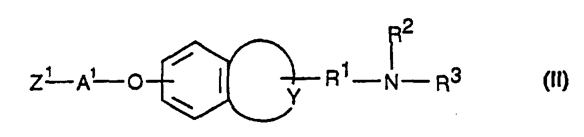 dans laquelle R¹, R², R³ et A¹ sont tels que définis dans la formule (I) et Z¹ représente une fonction réactive,
utiles en tant qu'intermédiaires de synthèse.

Les matières premières utilisées dans les procédés précédemment décrits sont soit commerciales ou connues dans l'état de la technique, soit aisément accessibles à l'homme du métier selon des procédés bien connus dans la littérature. On se référera plus particulièrement, pour les composés de formule générale (Il), aux descriptions du brevet EP 447 285 et de la demande de brevet EP 530 087.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité

Par exemple, les composés seront utilisés dans le traitement des dépressions saisonnières et des troubles du sommeil.

La présente invention a également pour objet les compositions pharmaceutiques contenant les produits de formule (I) ou le cas échéant un de leurs sels d'addition à une base pharmaceutiquement acceptable en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per- ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention, mais ne la limitent en aucune façon.

### PREPARATION 1 : N-{2-[7-(CARBOXYMETHYLOXY)NAPHT-1-YL]ETHYL}ACETAMIDE

### Stade A : N-{2-[7-(éthoxycarbonylméthyloxy)napht-1-yl]éthyl}acétamide

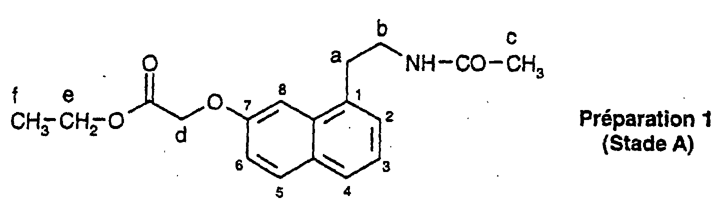

| **Réactifs :** | |
|---|---|
| N-[2-(7-hydroxy-napht-1-yl)éthyl]acétamide | 7 mmol (1,60 g) |
| Acétone anhydre | 30 cm³ |
| Carbonate de potassium | 14 mmol (1,93 g) |
| Bromoacétate d'éthyle | 10 mmol (1,67 g) |

### Mode opératoire :

Dissoudre le N-[2-(7-hydroxy-napht-1-yl)éthyl]acétamide dans l'acétone anhydre, ajouter le carbonate de potassium et laisser sous agitation à reflux durant une demi-heure. Ajouter le bromoacétate d'éthyle goutte à goutte à l'aide d'une ampoule à brome et laisser sous agitation à reflux durant trois heures. Laisser refroidir, essorer le précipité, évaporer le filtrat à sec, et recristalliser.

| **Caractéristiques :** | |
|---|---|
| Rendement | 80 % |
| Solvant de recristallisation | toluène/hexane (1/2) |
| Point de fusion | 95-97° C |
| Masse moléculaire | 315,355 g.mol ⁻¹pour C₁₂H₂₁NO₄ |

| **Microanalyse :** | | | |
|---|---|---|---|
| | % C | % H | % N |
| Calculé | 68,55 | 6,71 | 4,49 |
| Trouvé | 68,26 | 6,57 | 4,71 |

| **Infra-rouge :** | | |
|---|---|---|
| 3300 | cm⁻¹ | ν N-H |
| 2960-2860 | cm⁻¹ | ν C-H alkyles |
| 1735 | cm⁻¹ | ν C=O ester |
| 1620 | cm⁻¹ | ν C=O amide |

| **RMN (DMSO, d**_{**6**}**) 300 MHz :** | | | | |
|---|---|---|---|---|
| 1,25 | ppm | triplet | 3H | Hf J_{f-e} = 7,10 Hz |
| 1,85 | ppm | singulet | 3H | Hc |
| 3,15 | ppm | triplet | 2H | Ha J_{a-b} = 6,80 Hz |
| 3,35 | ppm | multiplet | 2H | Hb |
| 4,20 | ppm | quadruplet | 2H | He |
| 5,00 | ppm | singulet | 2H | Hd |
| 7,20-7,35 | ppm | massif | 3H | H₂, H₃, H₆ |
| 7,55 | ppm | doublet | 1H | H₈ J₈₋₆ = 2,15 Hz |
| 7,75 | ppm | doublet de doublet | 1H | H₄ J₄₋₃ = 7,40 Hz ; J₄₋₂ = 2,60 Hz |
| 7,85 | ppm | doublet | 1H | H₅J₅₋₆ = 9,00 Hz |
| 8,05 | ppm | triplet | 1H | N-H amide |

### Stade B : N-{2-[7-(carboxyméthyloxy)napht-1-yl]éthyl}acétamide

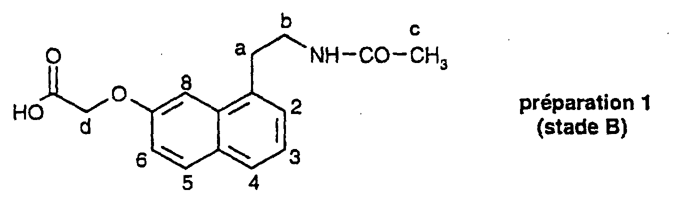

| **Réactifs :** | |
|---|---|
| N-{2-[7-(éthoxycarbonylméthyloxy)napht-1-yl]éthyl}acétamide | 5 mmol (1,57 g) |
| Solution aqueuse de soude à 10 % | 10 mmol (40 cm³) |

### Mode opératoire :

Dans une fiole, introduire le N-{2-[7-(éthoxycarbonylméthyloxy)-napht-1-yl]éthyl} acétamide et une solution aqueuse de soude à 10 % et laisser sous agitation à température ambiante jusqu'à dissolution. Refroidir dans un bain de glace et acidifier avec une solution d'acide chlorhydrique concentré. Essorer le précipité, laver à l'eau, sécher et recristalliser.

| **Caractéristiques :** | |
|---|---|
| Rendement | 70% |
| Solvant de recristallisation | éthanol 95°/eau (2/1) |
| Point de fusion | 181 - 184°C |
| Masse moléculaire | 296,311 g.mol⁻¹ pour C₁₆H₁₇NO₄ + 0,5 H₂O |

| **Microanalyse :** | | | |
|---|---|---|---|
| | % C | % H | % N |
| Calculé | 64,85 | 6,12 | 4,72 |
| Trouvé | 64,84 | 5,77 | 4,87 |

| **Infra-rouge :** | | |
|---|---|---|
| 3320 | cm⁻¹ | ν N-H amide |
| 2920-2860 | cm⁻¹ | ν C-H alkyles |
| 2500 | cm⁻¹ | ν CO₂ H |
| 1700 | cm⁻¹ | ν C=O acide |
| 1610 | cm⁻¹ | ν C=O amide |

| **RMN (DMSO, d**_{**6**}**) 300 MHz** | | | | |
|---|---|---|---|---|
| 1,80 | ppm | singulet | 3H | H_{c} |
| 3,10 | ppm | triplet | 2H | Hₐ J_{a-b} = 7,15 Hz |
| 3,35 | ppm | quadruplet | 2H | H_{b} |
| 4,90 | ppm | singulet | 2H | Hd |
| 7,30 | ppm | massif | 3H | H₂, H₃, H₆ |
| 7,55 | ppm | singulet | 1H | H₈ |
| 7,80 | ppm | doublet | 1H | H₄ J₄₋₃ = 7,15 Hz |
| 7,90 | ppm | doublet | 1H | H₅ J₅₋₆ = 8,60 Hz |
| 8,10 | ppm | signal | 1H | N-H |
| 13,00 | ppm | signal | 1H | O-H acide disparait avec D₂O |

### PREPARATION 2 : N-{2-[7-(PROPARGYLOXY)NAPHT-1-YL]ETHYL}ACETAMIDE

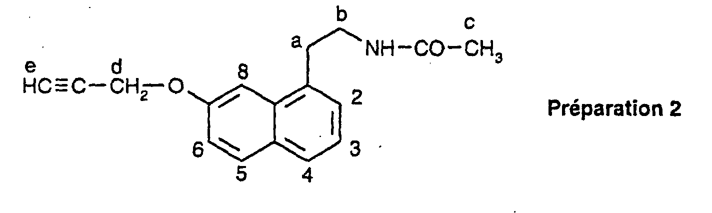

| **Réactifs :** | |
|---|---|
| N-[2-(7-hydroxy-napht-1-yl)éthyl]acétamide | 5 mmol (1,15 g) |
| hydrure de sodium | 18,75 mmol (0,45 g) |
| tosylate de l'alcool propargylique | 20 mmol |
| diméthylformamide | 30 cm³ |

### Mode opératoire :

Dans un ballon tricol, introduire le N-[2-(7-hydroxy-napht-1-yl)-éthyl]acétamide, le diméthylformamide, ajouter l'hydrure de sodium par petites fractions, laisser sous agitation durant deux heures sous azote à température ambiante. Additionner le tosylate de l'alcool propargylique goutte à goutte à l'aide d'une ampoule à brome ; laisser sous agitation durant une demi-heure, sous azote. Verser le mélange réactionnel dans l'eau sous agitation, extraire à l'acétate d'éthyle, laver à l'eau, sécher sur du chlorure de calcium, filtrer, évaporer à sec et recristalliser le résidu.

| **Caractéristiques :** | |
|---|---|
| Rendement | 59 % |
| Solvant de recristallisation | Hexane/toluène (2/1) |
| Point de fusion | 87 - 89° C |
| Masse moléculaire | 267,313 g.mol⁻¹ pour C₁₇H₁₇NO₂ |

| **Microanalyse :** | | | |
|---|---|---|---|
| | % C | % H | % N |
| Calculé | 76,37 | 6,41 | 5,24 |
| Trouvé | 76,12 | 6,30 | 5,33 |

| **Infra-rouge :** | | |
|---|---|---|
| 3270 | cm⁻¹ | ν N-H |
| 3200 | cm⁻¹ | ν C≡C-H |
| 2100 | cm⁻¹ | ν C≡C |
| 1620 | cm⁻¹ | ν C=O amide |

| **RMN (DMSO, d**_{**6**}**) 300 MHZ** | | | | |
|---|---|---|---|---|
| 1,85 | ppm | singulet | 3H | H_{c} |
| 3,15 | ppm | triplet | 2H | Hₐ J_{a-b} = 6,70 Hz |
| 3,30 | ppm | multiplet | 2H | H_{b} |
| 3,60 | ppm | singulet | 1H | Hₑ |
| 5,00 | ppm | singulet | 2H | H_{d} |
| 7,20-7,35 | ppm | massif | 3H | H₂, H₃, H₆ |
| 7,65 | ppm | singulet | 1H | H₈ |
| 7,75 | ppm | doublet | 1H | H₄ J₄₋₃ = 7,40 Hz |
| 7,85 | ppm | doublet | 1H | H₅ J₅₋₆ = 9,00 Hz |
| 8,05 | ppm | signal | 1H | NH amide |

En procédant de façon analogue, on obtient les préparations suivantes :

### PREPARATION 3 : N-{2-[7-(CARBOXYMETHYLOXY)NAPHT-1-YL]ETHYL} PROPIONAMIDE

### PREPARATION 4 : N-{2-[7-(CARBOXYMETHYLOXY)NAPHT-1-YL]ETHYL} BUTYRAMIDE

### PREPARATION 5 : N-{2-[7-(CARBOXYMETHYLOXY)NAPHT-1-YL]ETHYL} ISOBUTYRAMIDE

### PREPARATION 6 : N-{2-[7-(CARBOXYMETHYLOXY)NAPHT-1-YL]ETHYL} TRIFLUOROACETAMIDE

### PREPARATION 7 : N-{2-[7-(CARBOXYMETHYLOXY)NAPHT-1-YL]ETHYL} FORMAMIDE

### PREPARATION 8: N-{2-[7-(CARBOXYMETHYLOXY)NAPHT-1-YL]ETHYL} PENTANAMIDE

### PREPARATION 9 : N-{2-[7-(CARBOXYMETHYLOXY)NAPHT-1-YL]ETHYL} IODOACETAMIDE

### PREPARATION 10 : N-{2-[7-(CARBOXYMETHYLOXY)NAPHT-1-YL]ETHYL} CYCLOPROPANECARBOXAMIDE

### PREPARATION 11 : N-{2-[7-(CARBOXYMETHYLOXY)NAPHT-1-YL]ETHYL} CYCLOBUTANECARBOXAMIDE

### PREPARATION 12: N-{2-[7-(CARBOXYMETHYLOXY)NAPHT-1-YL]ETHYL} CYCLOPENTANECARBOXAMIDE

### PREPARATION 13 : N-{2-[7-(CARBOXYMETHYLOXY)NAPHT-1-YL]ETHYL} CYCLOHEXANECARBOXAMIDE

### PREPARATION 14: N-{2-[7-(CARBOXYMETHYLOXY)NAPHT-1-YL]ETHYL}PROP-1-ENYL-CARBOXAMIDE

### PREPARATION 15 : N-{2-[7-(CARBOXYMETHYLOXY)NAPHT-1-YL]ETHYL} N'-METHYLUREE

### PREPARATION 16 : N-{2-[7-(CARBOXYMETHYLOXY)NAPHT-1-YL]ETHYL} N'-ETHYLUREE

### PREPARATION 17 : N-{2-[7-(CARBOXYMETHYLOXY)NAPHT-1-YL]ETHYL} N'-n-PROPYLUREE

### PREPARATION 18 : N-{2-[7-(CARBOXYMETHYLOXY)NAPHT-1-YL]ETHYL} N'-CYCLOPROPYLUREE

### PREPARATION 19 : N-{2-[7-(CARBOXYMETHYLOXY)NAPHT-1-YL]ETHYL} N'-n-PROPYLTHIOUREE

### PREPARATION 20 : N-{2-[7-(CARBOXYMETHYLOXY)NAPHT-1-YL]ETHYL} N'-CYCLOPROPYLTHIOUREE

### PREPARATION 21 : N-{2-[7-(PROPARGYLOXY)NAPHT-1-YL]ETHYL} PROPIONAMIDE

### PREPARATION 22 : N-{2-[7-(PROPARGYLOXY)NAPHT-1-YL]ETHYL}BUTYRAMIDE

### PREPARATION 23 : N-{2-[7-(PROPARGYLOXY)NAPHT-1-YL]ETHYL}ISOBUTYRAMIDE

### PREPARATION 24 : N-{2-[7-(PROPARGYLOXY)NAPHT-1-YL]ETHYL}TRIFLUOROACETAMIDE

### PREPARATION 25 : N-{2-[7-(PROPARGYLOXY)NAPHT-1-YL]ETHYL}FORMAMIDE

### PREPARATION 26 : N-{2-[7-(PROPARGYLOXY)NAPHT-1-YL]ETHYL} PENTANAMIDE

### PREPARATION 27 : N-{2-[7-(PROPARGYLOXY)NAPHT-1-YL]ETHYL} IODOACETAMIDE

### PREPARATION 28 : N-{2-[7-(PROPARGYLOXY)NAPHT-1-YL]ETHYL}CYCLOPROPANECARBOXAMIDE

### PREPARATION 29 : N-{2-(7-(PROPARGYLOXY)NAPHT-1-YL]ETHYL} CYCLOBUTANECARBOXAMIDE

### PREPARATION 30 : N-{2-[7-(PROPARGYLOXY)NAPHT-1-YL]ETHYL} CYCLOPENTANECARBOXAMIDE

### PREPARATION 31: N-{2-[7-(PROPARGYLOXY)NAPHT-1-YL]ETHYL}CYCLOHEXANECARBOXAMIDE

### PREPARATION 32 : N-{2-[7-(PROPARGYLOXY)NAPHT-1-YL]ETHYL}PROP-1-ENYLCARBOXAMIDE

### PREPARATION 33: N-{2-[7-(PROPARGYLOXY)NAPHT-1-YL]ETHYL} N'-METHYLUREE

### PREPARATION 34: N-{2-[7-(PROPARGYLOXY)NAPHT-1-YL]ETHYL} N'-ETHYLUREE

### PREPARATION 35: N-{2-[7-(PROPARGYLOXY)NAPHT-1-YL]ETHYL} N'-PROPYLUREE

### PREPARATION 36: N-{2-[7-(PROPARGYLOXY)NAPHT-1-YL]ETHYL} N'-CYCLOPROPYLUREE

### PREPARATION 37: N-{2-[7-(PROPARGYLOXY)NAPHT-1-YL]ETHYL} N'-METHYLTHIOUREE

### PREPARATION 38: N-{2-[7-(PROPARGYLOXY)NAPHT-1-YL]ETHYL} N'-CYCLOPROPYLTHIOUREE

### PREPARATION 39: 2-[7H-8,9-DIHYDROPYRANO[3,2-e]INDOLYL]ETHYLAMINE

Ce composé est décrit dans J. Med. Chem. 1992, 35, p. 3625-3632.

### PREPARATION 40: N-[2-(8-ALLYL-7-HYDROXY-NAPHT-1-YL)ETHYL]ACETAMIDE

### Stade A : BROMHYDRATE DE 2-(7-HYDROXYNAPHT-1-YL)ETHYLAMINE

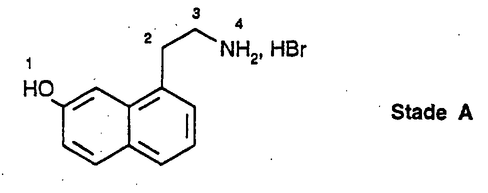

| **Réactifs :** | |
|---|---|
| Chlorhydrate de 2-(7-méthoxy napht-1-yl)éthylamine | 58 mmol (13,8 g) |
| Solution aqueuse de HBr à 47 % | 390 mmol (46 cm³) |

### Mode opératoire :

Dans un ballon de 250 cm³, on introduit le chlorhydrate d'éthylamine et la solution de HBr à 47 %. Le mélange est porté à reflux pendant 5 heures. Après refroidissement, le milieu réactionnel est filtré.

### Caractéristiques :

Masse moléculaire : 268,16 g pour C₁₂H₁₄BrNO
aspect : solide blanc
Point de fusion : 174-175°C
Rf : 0,72 éluant : Méthanol-ammoniaque à 28 % (4:1)
Rendement : 80 %
Solvant de recristallisation : acétate d'éthyle/hexane (1/3)

| **Infra-rouge :** | | |
|---|---|---|
| 3240-3460 | cm⁻¹ | ν OH |
| 3040-3100 | cm⁻¹ | ν C=C torsion |
| 2950-3060 | cm⁻¹ | ν CH |
| 2720-2480 | cm⁻¹ | ν NH₃+ |

| **RMN (DMSO, d6, δ) 80 MHz :** | | | | |
|---|---|---|---|---|
| 3,0-3,4 | ppm | massif | 4H | H₂, H₃ |
| 7,0-7,9 | ppm | massif | 6H | H aromatique |
| 8,1 | ppm | singulet | 3H | H₄ |
| 9,8 | ppm | singulet | 1H | H₁ |

| **Microanalyse :** | | | |
|---|---|---|---|
| | % C | % H | % N |
| Calculé | 53,75 | 5,26 | 5,22 |
| Trouvé | 53,84 | 5,30 | 5,32 |

### Stade B : N-[2-(7-HYDROXY NAPHT-1-YL) ETHYL]ACETAMIDE

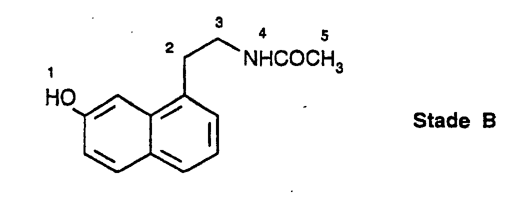

| **Réactifs :** | |
|---|---|
| Bromhydrate de la 2-(7-hydroxy napht-1-yl)éthylamine | 3,8 mmol (1,02 g) |
| Carbonate de sodium | 8,5 mmol (0,90 g) |
| Chlorure d'acétyle | 3,8 mmol (0,30 g) |

### Mode opératoire :

Dans une fiole de 50 cm³, dissoudre dans 5 cm³ d'eau le carbonate de sodium et sous agitation, ajouter le bromhydrate. Ajouter 20 cm³ d'acétate d'éthyle à la suspension obtenue, puis verser goutte à goutte le chlorure d'acétyle. Maintenir l'agitation pendant 30 minutes (la solution est limpide). Extraire la phase organique par de l'eau, puis par une solution aqueuse de HCI 1N, puis par de l'eau jusqu'à neutralité des eaux de lavage. Sécher la phase organique sur du sulfate de magnésium, la filtrer et sécher sous pression réduite.

### Caractéristiques :

Masse moléculaire : 229,27 g pour C₁₄H₁₅BrNO₂
aspect : solide blanc
Point de fusion : 125-126°C
Rf : 0,32 éluant : Acétone/Toluène/Cyclohexane (4/4/2)
Rendement : 60 %
Solvant de recristallisation : eau

| **Infrarouge :** | | |
|---|---|---|
| 3340 | cm⁻¹ | ν OH |
| 2980 | cm⁻¹ | ν CH |
| 1460 | cm⁻¹ | ν CH₃ |
| 1640 | cm⁻¹ | ν CO amide |

| **RMN (CDCl**_{**3**}**, δ) 80 MHz :** | | | | |
|---|---|---|---|---|
| 2,0 | ppm | singulet | 3H | H₅ |
| 3,2 | ppm | triplet | 2H | H₂ J₂₋₃ = 7,1 Hz |
| 3,6 | ppm | quintuplet | 2H | H₃ J₃₋₂ = 7,1 Hz ; J₃₋₄ = 7,1 Hz |
| 5,8 | ppm | signal | 1H | H₄ |
| 7,0-7,9 | ppm | massif | 6H | H aromatiques |
| 9,8 | ppm | singulet | 1H | H1 |

| **Microanalyse :** | | | |
|---|---|---|---|
| | % C | % H | % N |
| Calculé | 73,34 | 6,59 | 6,11 |
| Trouvé | 72,99 | 6,57 | 6,29 |

### Stade C : N-2-(7-ALLYLOXY NAPHT-1-YL) ETHYLACETAMIDE

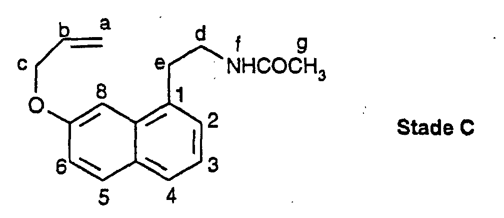

| **Réactifs :** | |
|---|---|
| N-[2-(7-hydroxy napht-1-yl)éthyl]acétamide | 20 mmol (5 g) |
| Carbonate de sodium | 50 mmol (6,63 g) |
| Bromure d'allyle | 30 mmol (3,63 g) |

### Mode opératoire :

Dissoudre le composé obtenu au stade précédent dans 100 cm³ d'acétone anhydre. Ajouter le carbonate de sodium et laisser sous agitation à reflux pendant 30 minutes. Ajouter goutte à goutte le bromure d'allyle. Laisser à reflux et sous agitation pendant 3 heures. Après refroidissement, filtrer le milieu réactionnel et sécher sous vide réduit le filtrat. L'huile obtenue est purifiée par chromatographie sur colonne.

### Caractéristiques :

Masse moléculaire : 269,33 g pour C₁₇H₁₉NO₂
aspect : huile
Rf : 0,19 éluant : Acétone/Toluène/Cyclohexane (2/3/5)
Rendement : 87 %

| **Infra-rouge :** | | |
|---|---|---|
| 3260 | cm⁻¹ | ν NH amide |
| 2920-2840 | cm⁻¹ | ν CH |
| 1635 | cm⁻¹ | ν CO amide |
| 1590 | cm⁻¹ | ν C=C |

| **RMN (CDCl**_{**3**}**, δ) 300 MHz:** | | | | | |
|---|---|---|---|---|---|
| 1,90 | ppm | singulet | 3H | Hg | |
| 3,20 | ppm | triplet | 2H | He | J_{e-d} = 7,00 Hz |
| 3,60 | ppm | quintuplet | 2H | Hd | |
| 4,70 | ppm | doublet | 2H | Hc | J_{c-b} = 5,28 Hz |
| 5,30 | ppm | doublet | 1H | Ha cis | J_{a-b} = 10,46 Hz |
| 5,50 | ppm | doublet | 1H | Ha trans | J_{a-b} = 17,30 Hz |
| 5,60 | ppm | signal | 1H | Hf | |
| 6,15 | ppm | multiplet | 1H | Hb | |
| 7,15 | ppm | doublet de doublet | 1H | H6 | Jₒᵣₜₕₒ = 8,90 J_{méta} = 2,30 |
| 7,25 | ppm | multiplet | 2H | H2,3 | |
| 7,40 | ppm | doublet | 1H | H8 | Jₒᵣₜₕₒ = 2,30 |
| 7,65 | ppm | multiplet | 1H | H3 | |
| 7,75 | ppm | doublet | 1H | H5 | Jₒᵣₜₕₒ = 8,30 |

| **Microanalyse :** | | | |
|---|---|---|---|
| | % C | % H | % N |
| Calculé | 75,80 | 7,11 | 5,20 |
| trouvé | 75,75 | 7,15 | 5,20 |

### Stade D : N-[2-(8-ALLYL 7-HYDROXY NAPHT-1-YL) ETHYL]ACETAMIDE

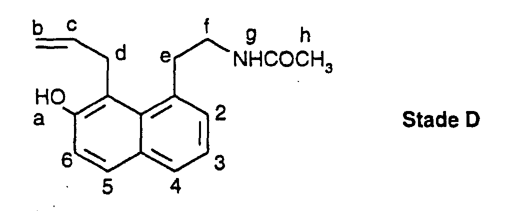

| **Réactifs :** | |
|---|---|
| N-[2-(7-allyloxy napht-1-yl) éthyl]acétamide | 7,4 mmol (2 g) |
| N,N-diméthylaniline | 7,4 mmol (10 cm³) |

### Mode opératoire :

Dissoudre le N-[2-(7-allyloxy napht-1-yl) éthyl]acétamide dans la N,N-diméthylaniline, porter le milieu réactionnel à reflux (200°C) pendant 2 heures. Après refroidissement, ajouter 20 cm³ d'éther et extraire la phase organique par une solution aqueuse de soude 10 % puis par de l'eau. La phase aqueuse est ensuite acidifiée par une solution aqueuse HCI 6N et laissée sous agitation pendant quelques minutes. Filtrer le précipité obtenu.

### Caractéristiques :

Masse moléculaire : 269,33 g.mol⁻¹ pour C₁₇H₁₉NO₂
aspect : solide jaune pâle
Rf : 0,38 éluant : Acétone/Toluène/Cyclohexane (4/4/2)
Point de fusion : 157-159°C
Rendement : 84 %
Solvant de recristallisation : cyclohexane

| **Infra-rouge :** | | |
|---|---|---|
| 3280 | cm⁻¹ | ν NH amide |
| 2860-3000 | cm⁻¹ | ν CH |
| 1600 | cm⁻¹ | ν CO amide |

| **RMN (DMSO, d6, δ) 300 MHz :** | | | | | |
|---|---|---|---|---|---|
| 1,83 | ppm | singulet | 3H | Hh | |
| 3,20 | ppm | signal | 2H | He | |
| 3,25 | ppm | signal | 2H | Hf | |
| 3,90 | ppm | signal | 2H | Hd | |
| 4,65 | ppm | doublet | 1H | Hb trans | J_{b-c} = 17,2 Hz |
| 4,95 | ppm | doublet | 1H | Hb cis | J_{b-c} = 8,8 Hz |
| 6,05 | ppm | multiplet | 1H | Hc | |
| 7,17 | ppm | signal | 1H | H6 | |
| 7,18 | ppm | signal | 1H | H3 | J₃₋₂ = 7,4 Hz ; J₃₋₄ = 4,33 Hz |
| 7,21 | ppm | signal | 1H | H2 | J₂₋₃ = 7,5 Hz |
| 7,65 | ppm | signal | 1H | H4 | J₄₋₃ = 7,4 Hz |
| 7,67 | ppm | signal | 1H | H5 | J₅₋₆ = 8,6 Hz |
| 8,08 | ppm | signal | 1H | Hg | |
| 9,60 | ppm | singulet | 1H | Ha échangeable dans D₂O | |

### PREPARATION 41 : N-[2-(8-ALLYL-7-HYDROXY-NAPHT-1-YL)ETHYL]N'-METHYLUREE

### EXEMPLE 1: 2,3-DIHYDRO-3-OXO-4-(2-ACETAMIDOETHYL)-1-NAPHTO[2,1-b] FURANNE

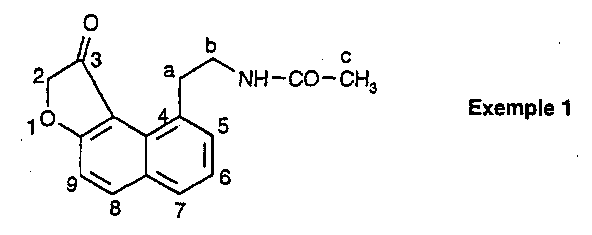

| **Réactifs :** | |
|---|---|
| N-{2-[7-(carboxyméthyloxyl)-napht-1-yl]éthyl}acétamide (préparation 1) | 10 mmol (2,9 g) |
| Acide polyphosphorique | 30 g |

### Mode opératoire :

Introduire dans un ballon de 100 cm³ à col rodé, le N-{2-[7-(carboxyméthyloxy)napht-1-yl]-éthyl}acétamide et l'acide polyphosphorique, agiter à l'aide d'un agitateur mécanique à 85° C durant deux heures et demie. Laisser sous agitation durant une heure. Verser dans de l'eau glacée.
Extraire par l'acétate d'éthyle, laver la phase organique deux fois par une solution aqueuse de carbonate de sodium à 10 %, puis laver à l'eau, sécher sur le chlorure de calcium, filtrer et évaporer à sec. Le produit est purifié sur colonne avec gel de silice 60 Å en utilisant l'éluant acétone/toluène (1/1).

| **Caractéristiques :** | |
|---|---|
| Rendement | 32 % |
| Solvant de recristallisation | Hexane/toluène (2/1) |
| Point de fusion | 157 - 158° C |
| Masse moléculaire | 269,287 g.mol⁻¹ pour C₁₆H₁₅NO₃ |

| **Microanalyse :** | | | |
|---|---|---|---|
| | % C | % N | % N |
| Calculé | 71,35 | 5,61 | 5,20 |
| Trouvé | 71,33 | 5,46 | 5,17 |

| **Infra-rouge :** | | |
|---|---|---|
| 3270 | cm⁻¹ | ν N-H amide |
| 2920-2860 | cm⁻¹ | ν C-H alkyle |
| 1685 | cm⁻¹ | ν C=O cétonique |
| 1610 | cm⁻¹ | ν C=O amide |

| **RMN (dMSO, d**_{**6**}**) 300 MHz :** | | | | |
|---|---|---|---|---|
| 1,75 | ppm | singulet | 3H | H_{c} |
| 3,25 | ppm | quadruplet | 2H | H_{b} |
| 3,60 | ppm | triplet | 2H | Hₐ, J_{a-b} = 6,60 Hz |
| 7,45 | ppm | massif | 3H | H₅, H₆, H₉ |
| 7,75 | ppm | signal | 1H | N-H |
| 7,85 | ppm | doublet | 1H | H₇, J₇₋₆ = 7,40 Hz |
| 8,30 | ppm | doublet | 1H | H₈, J₈₋₉ = 9,00 Hz |

### EXEMPLE 2 : 2,3-DIHYDRO-3-HYDROXY-4-(2-ACETAMIDOETHYL)-1-NAPHTO[2,1-b] FURANNE

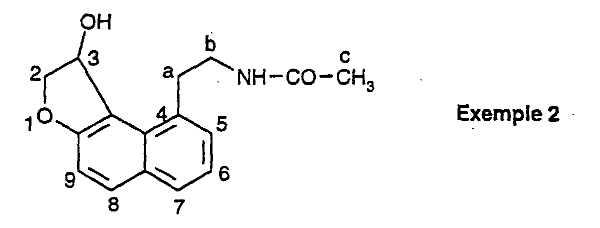

| **Réactifs :** | |
|---|---|
| 2,3-dihydro-3-oxo-4-(2-acétamidoéthyl)-1-naphto[2,1-b]furanne (exemple 1) | 5 mmol (1,35 g) |
| Méthanol | 30 cm³ |
| Borohydrure de sodium | 10 mmol (0,32 g) |

### Mode opératoire :

Introduire dans une fiole à col rodé de 100 cm³, le 2,3-dihydro-3-oxo-4-(2-acétamidoéthyl)-1-naphto[2,1-b]furanne et le méthanol, ajouter le borohydrure de sodium (5 mmol) par petites fractions et laisser sous agitation. Au bout de deux heures, ajouter 5 mmol de borohydrure de sodium par petites fractions et laisser sous agitation toute la nuit à température ambiante. Evaporer à sec, reprendre par l'eau, acidifier par une solution d'acide chlorhydrique 6N. Essorer le précipité, le laver à l'eau jusqu'à neutralité de l'eau de lavage, le sécher et le recristalliser dans le toluène.

| **Caractéristiques :** | |
|---|---|
| Rendement | 51 % |
| Point de fusion | 153-156°C |
| Masse moléculaire | 271,303 g.mol⁻¹ pour C₁₆H₁₇NO₃ |

| **Microanalyse :** | | | |
|---|---|---|---|
| | % C | % H | % N |
| Calculé | 70,82 | 6,31 | 5,16 |
| Trouvé | 70,61 | 6,28 | 5,04 |

| **Infra-rouge :** | | |
|---|---|---|
| 3250 | cm⁻¹ | ν O-H et N-H |
| 1620 | cm⁻¹ | ν C=O amide |

| **RMN (DMSO, d**_{**6**}**) 300 MHz :** | | | | |
|---|---|---|---|---|
| 1,80 | ppm | singulet | 3H | H_{c} |
| 3,00-3,65 | ppm | massif | 4H | Hₐ, H_{b} |
| 4,50 | ppm | multiplet | 2H | H₂ |
| 5,60 | ppm | doublet | 1H | OH disparait avec D₂O J = 7,00 |
| 5,70 | ppm | multiplet | 1H | H₃ |
| 7,20-7,35 | ppm | massif | 3H | H₅, H₆, H₉ |
| 7,75 | ppm | doublet | 1H | H₇ J₇₋₆ = 7,85 Hz |
| 7,90 | ppm | doublet | 1H | H₈ J₈₋₉ = 8,80 Hz |
| 8,05 | ppm | signal | 1H | N-H amide |

### EXEMPLE 3 : 4-(2-ACETAMIDOETHYL)-1-NAPHTO[2,1-b]FURANNE

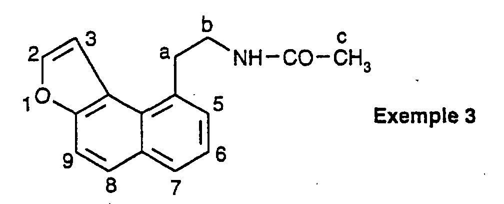

| **Réactifs :** | |
|---|---|
| 2,3-dihydro-3-oxo-4-(2-acétamidoéthyl)-1-naphto[2,1-b]furanne (exemple 1) | 5 mmol (1,38 g) |
| Méthanol | 30 cm³ |
| Borohydrure de sodium | 20 mmol (0,76 g) |

### Mode opératoire :

Introduire dans un ballon de 100 cm³, le 2,3-dihydro-3-oxo-4-(2-acétamidoéthyl)-1-naphto[2,1-b]furanne et le méthanol, ajouter 10 mmol de borohydrure de sodium par petites fractions sous agitation.
Au bout de deux heures, ajouter 10 mmol de borohydrure de sodium par petites fractions sous agitation. Laisser sous agitation à température ambiante, acidifier par une solution d'acide chlorhydrique 6N, évaporer le méthanol, reprendre par l'eau, essorer le précipité, le laver à l'eau jusqu'à neutralité de l'eau de lavage, le sécher et le recristalliser dans le mélange toluène/hexane.

| | |
|---|---|
| Rendement | 85 % |
| Point de fusion | 142 - 144° C |
| Masse moléculaire | 253,287 g.mol⁻¹ pour C₁₆H₁₅NO₂ |

| **Microanalyse :** | | | |
|---|---|---|---|
| | %C | %H | %N |
| Calculé | 75,80 | 5,96 | 5,53 |
| Trouvé | 75,57 | 5,97 | 5,47 |

| **Infra-rouge :** | | |
|---|---|---|
| 3240 | cm⁻¹ | ν N-H amide |
| 1625 | cm⁻¹ | ν C=O amide |

| **RMN (DMSO, d**_{**6**}**) 300 MHz** | | | | |
|---|---|---|---|---|
| 1,85 | ppm | singulet | 3H | H_{c} |
| 3,40 | ppm | multiplet | 4H | Hₐ, H_{b} |
| 7,50 | ppm | multiplet | 2H | H₃, H₉ |
| 7,80-7,95 | ppm | massif | 4H | H₅, H₆, H₇, H₈ |
| 8,20 | ppm | multiplet | 2H | H₂, NH |

### EXEMPLE 4: 5-(2-ACETAMIDOETHYL)-2H-1-NAPHTO[2,1-b]PYRANNE

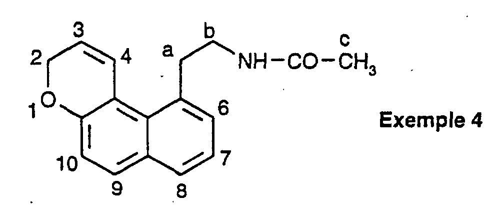

| **Réactifs :** | |
|---|---|
| N-{2-[7-(propargyloxy)-napht-1-yl]éthyl}acétamide (préparation 2) | 10 mmol (2,67 g) |
| Triéthylène glycol | 40 cm³ |

### Mode opératoire :

Introduire dans un ballon à bicol le N-{2-[7-(propargyloxy)-napht-1-yl]éthyl} acétamide et le triéthylène glycol. Chauffer à 160°-170° C sous azote et sous agitation durant cinq heures. Verser le mélange réactionnel dans l'eau glacée, extraire à l'acétate d'éthyle, laver à l'eau, sécher sur le chlorure de calcium, filtrer et évaporer à sec.
Le produit est purifié sur colonne de silice 60 Å avec un éluant acétone/toluène (1/1).

| **Caractéristiques :** | |
|---|---|
| Rendement | 23 % |
| Solvant de recristallisation | toluène/hexane |
| Point de fusion | se décompose à 113° C |
| Masse moléculaire | 267,313 g.mol⁻¹ pour C₁₇H₁₇NO₂ |

| **Microanalyse :** | | | |
|---|---|---|---|
| | %C | %H | % N |
| Calculé | 76,37 | 6,41 | 5,24 |
| Trouvé | 76,16 | 6,40 | 5,52 |

| **Infra-rouge :** | | |
|---|---|---|
| 3250 | cm⁻¹ | ν N-H |
| 2960-2840 | cm⁻¹ | ν C-H alkyles |
| 1630 | cm⁻¹ | ν C=O amide |

| **RMN (DMSO, d**_{**6**}**) 300 MHz** | | | | |
|---|---|---|---|---|
| 1,80 | ppm | singulet | 3H | H_{c} |
| 3,20 | ppm | triplet | 2H | Hₐ J_{a-b} = 6,80 Hz |
| 3,40 | ppm | multiplet | 2H | H_{b} |
| 4,65 | ppm | doublet | 2H | H₂ J₂₋₃ = 4,30 Hz |
| 5,90 | ppm | multiplet | 1H | H₃ |
| 7,10 | ppm | doublet | 1H | H₄ J₄₋₃ = 8,80 Hz |
| 7,30 | ppm | massif | 3H | H₆,H₇,H₁₀ |
| 7,70 | ppm | doublet | 1H | H₈ J₈₋₇ = 7,50 Hz |
| 7,80 | ppm | doublet | 1H | H₉ J₉₋₁₀ = 9,80 Hz |
| 8,10 | ppm | signal | 1H | N-H amide |

### EXEMPLE 5 : 3,4,5,6,7,8-HEXAHYDRO-5-(2-ACETAMIDOETHYL)-2H-1-NAPHTO[2,1-b] PYRANNE

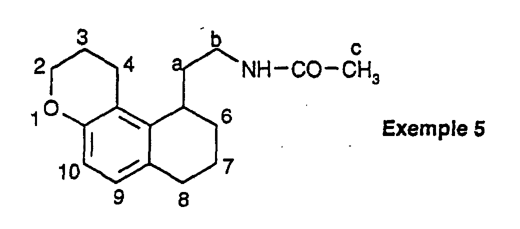

| **Réactifs :** | |
|---|---|
| 5-(2-acétamidoéthyl)-2H-1-naphto[2,1-b]pyranne (exemple 4) | 2 mmol (5,34 mg) |
| Méthanol | 25 cm³ |
| Nickel de Raney | quelques mg |

### Mode opératoire :

Dissoudre le 5-(2-acétamidoéthyl)-2H-1-naphto[2,1-b]pyranne dans le méthanol, ajouter le nickel de Raney et agiter sous atmosphère d'hydrogène à pression ordinaire à température ambiante durant six heures. Filtrer. Evaporer à sec et recristalliser.

| **Caractéristiques :** | |
|---|---|
| Rendement | 55 % |
| Solvant de recristallisation | toluène |
| Point de fusion | 117 - 118° C |
| Masse moléculaire | 273,361g.mol⁻¹ pour C₁₇H₂₃NO₂ |

| **Microanalyse :** | | | |
|---|---|---|---|
| | % C | %H | %N |
| Calculé | 74,68 | 8,48 | 5,12 |
| Trouvé | 74,46 | 8,39 | 5,16 |

| **Infra-rouge :** | | |
|---|---|---|
| 3240 | cm⁻¹ | ν N-H amide |
| 2980-2800 | cm⁻¹ | ν C-H alkyles |
| 1610 | cm⁻¹ | ν C=O amide |

| **RMN (DMSO, d**_{**6**}**) 80 MHz** | | | | |
|---|---|---|---|---|
| 1,30-2,15 | ppm | massif | 11H | Hₐ, H_{c}, H₃, H₆, H₇ |
| 2,35-2,80 | ppm | massif | 5H | H₄, H₅, H₈ |
| 3,20 | ppm | multiplet | 2H | H_{b} |
| 4,00 | ppm | multiplet | 2H | H₂ |
| 6,50 | ppm | doublet | 1H | H₁₀ J₁₀₋₉ = 9,20 Hz |
| 6,75 | ppm | doublet | 1H | H₉ J₉₋₁₀ = 9,20 Hz |
| 7,90 | ppm | signal | 1H | N-H amide |

### EXEMPLE 6 : 3,4-DIHYDRO-5-(2-ACETAMIDOETHYL)-2H-1-NAPHTO[2,1-b]PYRANNE

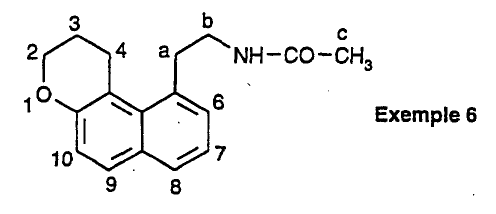

| **Réactifs :** | |
|---|---|
| 5-(2-acétamidoéthyl)-2H-1-naphto[2,1-b]pyranne (exemple 4) | 2 mmol (534 mg) |
| Méthanol | 80 cm³ |
| Magnésium | 80 mmol (1,35 g) |

### Mode opératoire :

Dissoudre le 5-(2-acétamidoéthyl)-2H-1-naphto[2,1-b]pyranne dans le méthanol, refroidir à l'aide d'un bain de glace-sel. Ajouter le magnésium par petites fractions et laisser sous agitation à température ambiante durant 16 heures. Ajouter 30 cm³ d'une solution d'acide chlorhydrique 6N petit à petit, sous agitation. Laisser refroidir, extraire à l'éther, laver la phase organique à l'eau, sécher sur le sulfate de magnésium, filtrer et évaporer à sec.

| **Caractéristiques :** | |
|---|---|
| Rendement | 42 % |
| Solvant de recristallisation | éther/éther de pétrole |
| Point de fusion | 137 - 139° C |
| Masse moléculaire | 291,849 g.mol⁻¹ pour C₁₇H₁₉NO₂ + 1,25 H₂O |

| **Microanalyse :** | | | |
|---|---|---|---|
| | % C | % H | % N |
| Calculé | 69,95 | 6,99 | 4,79 |
| Trouvé | 70,00 | 6,63 | 4,75 |

| **Infra-rouge :** | | |
|---|---|---|
| 3240 | cm⁻¹ | ν N-H amide |
| 2980-2800 | cm⁻¹ | ν C-H alkyles |
| 1610 | cm⁻¹ | ν C=O amide |

| **RMN (DMSO, d**_{**6**}**) 300 MHz :** | | | | |
|---|---|---|---|---|
| 1,50-2,10 | ppm | massif | 5H | H₃, H_{c} |
| 3,10-3,85 | ppm | massif | 6H | Hₐ, H_{b}, H₄ |
| 3,95 | ppm | multiplet | 2H | H₂ |
| 7,15-7,30 | ppm | massif | 3H | H₆ H₇ ,H₁₀ |
| 7,65 | ppm | doublet | 1H | H₈ J₈₋₇ = 7,45 Hz |
| 7,80 | ppm | doublet | 1H | H₉ J₉₋₁₀ = 9,90 Hz |
| 8,10 | ppm | signal | 1H | N-H |

### EXEMPLES 7 A 114 :

En procédant comme dans les exemples 1 à 6 mais en utilisant les préparations appropriées, on obtient les composés des exemples suivants.

### EXEMPLE 7 : 2,3-DIHYDRO-3-OXO-4-(2-PROPIONAMIDOETHYL)-1-NAPHTO[2,1-b] FURANNE

### EXEMPLE 8: 2,3-DIHYDRO-3-OXO-4-(2-BUTYRAMIDOETHYL)-1-NAPHTO[2,1-b] FURANNE

### EXEMPLE 9: 2,3-DIHYDRO-3-OXO-4-(2-ISOBUTYRAMIDOETHYL)-1-NAPHTO[2,1-b] FURANNE

### EXEMPLE 10: 2,3-DIHYDRO-3-OXO-4-(2-TRIFLUOROACETAMIDOETHYL)-1-NAPHTO [2,1-b]FURANNE

### EXEMPLE 11: 2,3-DIHYDRO-3-OXO-4-(2-FORMAMIDOETHYL)-1-NAPHTO[2,1-b] FURANNE

### EXEMPLE 12: 2,3-DIHYDRO-3-OXO-4-(2-PENTANAMIDOETHYL)-1-NAPHTO[2,1-b] FURANNE

### EXEMPLE 13 : 2,3-DIHYDRO-3-OXO-4-[2-(IODOACETAMIDO)ETHYL]-1-NAPHTO [2,1-b]FURANNE

### EXEMPLE 14: 2,3-DIHYDRO-3-OXO-4-[2-(CYCLOPROPANECARBOXAMIDO)ETHYL]-1-NAPHTO[2,1-b]FURANNE

### EXEMPLE 15: 2,3-DIHYDRO-3-OXO-4-[2-(CYCLOBUTANECARBOXAMIDO)ETHYL]-1-NAPHTO[2,1-b]FURANNE

### EXEMPLE 16: 2,3-DIHYDRO-3-OXO-4-[2-(CYCLOPENTANECARBOXAMIDO)ETHYL]-1-NAPHTO[2,1-b]FURANNE

### EXEMPLE 17: 2,3-DIHYDRO-3-OXO-4-[2-(CYCLOHEXANECARBOXAMIDO)ETHYL]-1-NAPHTO[2,1-b]FURANNE

### EXEMPLE 18: 2,3-DIHYDRO-3-OXO-4-[2-(PROP-1-ENYLCARBOXAMIDO)ETHYL]-1-NAPHTO [2,1-b]FURANNE

### EXEMPLE 19 : 2,3-DIHYDRO-3-HYDROXY-4-(2-PROPIONAMIDOETHYL)-1-NAPHTO [2,1-b]FURANNE

### EXEMPLE 20 : 2,3-DIHYDRO-3-HYDROXY-4-(2-BUTYRAMIDOETHYL)-1-NAPHTO [2,1-b]FURANNE

### EXEMPLE 21 : 2,3-DIHYDRO-3-HYDROXY-4-(2-ISOBUTYRAMIDOETHYL)-1-NAPHTO [2,1-b]FURANNE

### EXEMPLE 22 : 2,3-DIHYDRO-3-HYDROXY-4-(2-TRIFLUOROACETAMIDOETHYL)-1-NAPHTO[2,1-b]FURANNE

### EXEMPLE 23 : 2,3-DIHYDRO-3-HYDROXY-4-(2-FORMAMIDOETHYL)-1-NAPHTO[2,1-b] FURANNE

### EXEMPLE 24 : 2,3-DIHYDRO-3-HYDROXY-4-(2-PENTANAMIDOETHYL)-1-NAPHTO [2,1-b]FURANNE

### EXEMPLE 25 : 2,3-DIHYDRO-3-HYDROXY-4-[2-(IODOACETAMIDO)ETHYL]-1-NAPHTO[2,1-b]FURANNE

### EXEMPLE 26: 2,3-DIHYDRO-3-HYDROXY-4-[2-(CYCLOPROPANECARBOXAMIDO) ETHYL]-1-NAPHTO[2,1-b]FURANNE

### EXEMPLE 27: 2,3-DIHYDRO-3-HYDROXY-4-[2-(CYCLOBUTANECARBOXAMIDO) ETHYL]-1-NAPHTO[2,1-b]FURANNE

### EXEMPLE 28: 2,3-DIHYDRO-3-HYDROXY-4-[2-(CYCLOPENTANECARBOXAMIDO) ETHYL]-1-NAPHTO[2,1-b]FURANNE

### EXEMPLE 29: 2,3-DIHYDRO-3-HYDROXY-4-[2-(CYCLOHEXANECARBOXAMIDO) ETHYL]-1-NAPHTO[2,1-b]FURANNE

### EXEMPLE 30: 2,3-DIHYDRO-3-HYDROXY-4-[2-(PROP-1-ENYLCARBOXAMIDO) ETHYL]-1-NAPHTO[2,1-b]FURANNE

### EXEMPLE 31: 4-(2-PROPIONAMIDOETHYL)-1-NAPHTO[2,1-b]FURANNE

### EXEMPLE 32 : 4-(2-BUTYRAMIDOETHYL)-1-NAPHTO[2,1-b]FURANNE

### EXEMPLE 33 : 4-(2-ISOBUTYRAMIDOETHYL)-1-NAPHTO[2,1-b]FURANNE

### EXEMPLE 34 : 4-(2-TRIFLUOROACETAMIDOETHYL)-1-NAPHTO[2,1-b]FURANNE

### EXEMPLE 35 : 4-(2-FORMAMIDOETHYL)-1-NAPHTO[2,1-b]FURANNE

### EXEMPLE 36 : 4-(2-PENTANAMIDOETHYL)-1-NAPHTO[2,1-b]FURANNE

### EXEMPLE 37 : 4-[2-(IODOACETAMIDO)ETHYL]-1-NAPHTO[2,1-b]FURANNE

### EXEMPLE 38: 4-[2-(CYCLOPROPANECARBOXAMIDO)ETHYL]-1-NAPHTO[2,1-b] FURANNE

### EXEMPLE 39: 4-[2-(CYCLOBUTANECARBOXAMIDO)ETHYL]-1-NAPHTO[2,1-b] FURANNE

### EXEMPLE 40: 4-[2-(CYCLOPENTANECARBOXAMIDO)ETHYL]-1-NAPHTO[2,1-b] FURANNE

### EXEMPLE 41: 4-[2-(CYCLOHEXANECARBOXAMIDO)ETHYL]-1-NAPHTO[2,1-b] FURANNE

### EXEMPLE 42: 4-[2-(PROP-1-ENYLCARBOXAMIDO)ETHYL]-1-NAPHTO[2,1-b] FURANNE

### EXEMPLE 43 : 5-(2-PROPIONAMIDOETHYL)-2H-1-NAPHTO[2,1-b]PYRANNE

### EXEMPLE 44 : 5-(2-BUTYRAMIDOETHYL)-2H-1-NAPHTO[2,1-b]PYRANNE

### EXEMPLE 45 : 5-(2-ISOBUTYRAMIDOETHYL)-2H-1-NAPHTO[2,1-b]PYRANNE

### EXEMPLE 46 : 5-(2-TRIFLUOROACETAMIDOETHYL)-2H-1-NAPHTO[2,1-b]PYRANNE

### EXEMPLE 47 : 5-(2-FORMAMIDOETHYL)-2H-1-NAPHTO[2,1-b]PYRANNE

### EXEMPLE 48 : 5-(2-PENTANAMIDOETHYL)-2H-1-NAPHTO[2,1-b]PYRANNE

### EXEMPLE 49 : 5-[2-(IODOACETAMIDO)ETHYL]-2H-1-NAPHTO[2,1-b]PYRANE

### EXEMPLE 50 : 5-[2-(CYCLOPROPANECARBOXAMIDO)ETHYL]-2H-1-NAPHTO[2,1-b] PYRANNE

### EXEMPLE 51 : 5-[2-(CYCLOBUTANECARBOXAMIDO)ETHYL]-2H-1-NAPHTO[2,1-b] PYRANNE

### EXEMPLE 52 : 5-[2-(CYCLOPENTANECARBOXAMIDO)ETHYL]-2H-1-NAPHTO[2,1-b] PYRANNE

### EXEMPLE 53 : 5-[2-(CYCLOHEXANECARBOXAMIDO)ETHYL]-2H-1-NAPHTO[2,1-b] PYRANNE

### EXEMPLE 54 : 5-[2-(PROP-1-ENYLCARBOXAMIDO)ETHYL]-2H-1-NAPHTO[2,1-b] PYRANNE

### EXEMPLE 55 : 3,4,5,6,7,8-HEXAHYDRO-5-(2-PROPIONAMIDOETHYL)-2H-1-NAPHTO[2,1-b]PYRANNE

### EXEMPLE 56 : 3,4,5,6,7,8-HEXAHYDRO-5-(2-BUTYRAMIDOETHYL)-2H-1-NAPHTO [2,1-b]PYRANNE

### EXEMPLE 57 : 3,4,5,6,7,8-HEXAHYDRO-5-(2-ISOBUTYRAMIDOETHYL)-2H-1-NAPHTO [2,1-b]PYRANNE

### EXEMPLE 58 : 3,4,5,6,7,8-HEXAHYDRO-5-(2-TRIFLUOROACETAMIDOETHYL)-2H-1- NAPHTO[2,1-b]PYRANNE

### EXEMPLE 59 : 3,4,5,6,7,8-HEXAHYDRO-5-(2-FORMAMIDOETHYL)-2H-1-NAPHTO [2,1-b]PYRANNE

### EXEMPLE 60 : 3,4,5,6,7,8-HEXAHYDRO-5-(2-PENTANAMIDOETHYL)-2H-1-NAPHTO [2,1-b]PYRANNE

### EXEMPLE 61 : 3,4,5,6,7,8-HEXAHYDRO-5-[2-(IODOACETAMIDO)ETHYL]-2H-1-NAPHTO[2,1-b]PYRANNE

### EXEMPLE 62 : 3,4,5,6,7,8-HEXAHYDRO-5-[2-(CYCLOPROPANECARBOXAMIDO) ETHYL]-2H-1-NAPHTO[2,1-b]PYRANNE

### EXEMPLE 63 : 3,4,5,6,7,8-HEXAHYDRO-5-[2-(CYCLOBUTANECARBOXAMIDO) ETHYL]-2H-1-NAPHTO[2,1-b]PYRANNE

### EXEMPLE 64 : 3,4,5,6,7,8-HEXAHYDRO-5-[2-(CYCLOPENTANECARBOXAMIDO) ETHYL]-2H-1-NAPHTO[2,1-b]PYRANNE

### EXEMPLE 65 : 3,4,5,6,7,8-HEXAHYDRO-5-[2-(CYCLOHEXANECARBOXAMIDO) ETHYL]-2H-1-NAPHTO[2,1-b]PYRANNE

### EXEMPLE 66 : 3,4,5,6,7,8-HEXAHYDRO-5-[2-(PROP-1-ENYLCARBOXAMIDO)ETHYL]-2H-1-NAPHTO[2,1-b]PYRANNE

### EXEMPLE 67 : 3,4-DIHYDRO-5-(2-PROPIONAMIDOETHYL)-2H-1-NAPHTO[2,1-b] PYRANNE

### EXEMPLE 68 : 3,4-DIHYDRO-5-(2-BUTYRAMIDOETHYL)-2H-1-NAPHTO[2,1-b] PYRANNE

### EXEMPLE 69 : 3,4-DIHYDRO-5-(2-ISOBUTYRAMIDOETHYL)-2H-1-NAPHTO[2,1-b] PYRANNE

### EXEMPLE 70: 3,4-DIHYDRO-5-(2-TRIFLUOROACETAMIDOETHYL)-2H-1-NAPHTO [2,1-b]PYRANNE

### EXEMPLE 71 : 3,4-DIHYDRO-5-(2-FORMAMIDOETHYL)-2H-1-NAPHTO[2,1-b] PYRANNE

### EXEMPLE 72: 3,4-DIHYDRO-5-(2-PENTANAMIDOETHYL)-2H-1-NAPHTO[2,1-b] PYRANNE

### EXEMPLE 73 : 3,4-DIHYDRO-5-[2-(IODOACETAMIDO)ETHYL]-2H-1-NAPHTO[2,1-b] PYRANNE

### EXEMPLE 74 : 3,4-DIHYDRO-5-[2-(CYCLOPROPANECARBOXAMIDO)ETHYL]-2H-1-NAPHTO[2,1-b]PYRANNE

### EXEMPLE 75 : 3,4-DIHYDRO-5-[2-(CYCLOBUTANECARBOXAMIDO)ETHYL]-2H-1-NAPHTO [2,1-b]PYRANNE

### EXEMPLE 76 : 3,4-DIHYDRO-5-[2-(CYCLOPENTANECARBOXAMIDO)ETHYL]-2H-1-NAPHTO[2,1-b]PYRANNE

### EXEMPLE 77 : 3,4-DIHYDRO-5-[2-(CYCLOHEXANECARBOXAMIDO)ETHYL]-2H-1-NAPHTO [1,2-b]PYRANNE

### EXEMPLE 78 : 3,4-DIHYDRO-5-[2-(PROP-1-ENYLCARBOXAMIDO)ETHYL]-2H-1-NAPHTO[2,1-b] PYRANNE

### EXEMPLE 79: N-[2-(2,3-DIHYDRO-3-OXO-1-NAPHTO[2,1-b]FURANN-4-YL)ETHYL] N'-METHYLUREE

### EXEMPLE 80: N-[2-(2,3-DIHYDRO-3-OXO-1-NAPHTO[2,1-b]FURANN-4-YL)ETHYL] N'-ETHYLUREE

### EXEMPLE 81 : N-[2-(2,3-DIHYDRO-3-OXO-1-NAPHTO[2,1-b]FURANN-4-YL)ETHYL] N'-n-PROPYLUREE

### EXEMPLE 82 : N-[2-(2,3-DIHYDRO-3-OXO-1-NAPHTO[2,1-b]FURANN-4-YL)ETHYL] N'-CYCLOPROPYLUREE

### EXEMPLE 83 : N-[2-(2,3-DIHYDRO-3-OXO-1-NAPHTO[2,1-b]FURANN-4-YL)ETHYL] N'-n-PROPYLTHIOUREE

### EXEMPLE 84 : N-[2-(2,3-DIHYDRO-3-OXO-1-NAPHTO[2,1-b]FURANN-4-YL)ETHYL] N'-CYCLOPROPYLTHIOUREE

### EXEMPLE 85 : N-[2-(2,3-DIHYDRO-3-HYDROXY-1-NAPHTO[2,1-b]FURANN-4-YL) ETHYL] N'-METHYLUREE

### EXEMPLE 86 : N-[2-(2,3-DIHYDRO-3-HYDROXY-1-NAPHTO[2,1-b]FURANN-4-YL) ETHYL]N'-ETHYLUREE

### EXEMPLE 87 : N-[2-(2,3-DIHYDRO-3-HYDROXY-1-NAPHTO[2,1-b]FURANN-4-YL) ETHYL] N'-PROPYLUREE

### EXEMPLE 88 : N-[2-(2,3-DIHYDRO-3-HYDROXY-1-NAPHTO[2,1-b]FURANN-4-YL) ETHYL] N'-CYCLOPROPYLUREE

### EXEMPLE 89 : N-[2-(2,3-DIHYDRO-3-HYDROXY-1-NAPHTO[2,1-b]FURANN-4-YL) ETHYL] N'-PROPYLTHIOUREE

### EXEMPLE 90 : N-[2-(2,3-DIHYDRO-3-HYDROXY-1-NAPHTO[2,1-b]FURANN-4-YL) ETHYL] N'-CYCLOPROPYLTHIOUREE

### EXEMPLE 91 : N-[2-(1-NAPHTO[2,1-b]FURANN-4-YL)ETHYL]N'-METHYLUREE

### EXEMPLE 92 : N-[2-(1-NAPHTO[2,1-b]FURANN-4-YL)ETHYL] N'-ETHYLUREE

### EXEMPLE 93 : N-[2-(1-NAPHTO[2,1-b]FURANN-4-YL)ETHYL] N'-PROPYLUREE

### EXEMPLE 94 : N-[2-(1-NAPHTO[2,1-b]FURANN-4-YL)ETHYL]N'-CYCLOPROPYLUREE

### EXEMPLE 95 : N-[2-(1-NAPHTO[2,1-b]FURANN-4-YL)ETHYL] N'-PROPYLTHIOUREE

### EXEMPLE 96 : N-[2-(1-NAPHTO[2,1-b]FURANN-4-YL)ETHYL] N'-CYCLOPROPYLTHIOUREE

### EXEMPLE 97 : N-[2-(2H-1-NAPHTO[2,1-b]PYRANN-5-YL)ETHYL] N'-METHYLUREE

### EXEMPLE 98 : N-[2-(2H-1-NAPHTO[2,1-b]PYRANN-5-YL)ETHYL]N'-ETHYLUREE

### EXEMPLE 99 : N-[2-(2H-1-NAPHTO[2,1-b]PYRANN-5-YL)ETHYL] N'-n-PROPYLUREE

### EXEMPLE 100 : N-[2-(2H-1-NAPHTO[2,1-b]PYRANN-5-YL)ETHYL] N'-CYCLOPROPYLUREE

### EXEMPLE 101 : N-[2-(2H-1-NAPHTO[2,1-b]PYRANN-5-YL)ETHYL] N'-PROPYL THIOUREE

### EXEMPLE 102 : N-[2-(2H-1-NAPHTO[2,1-b]PYRANN-5-YL)ETHYL] N'-CYCLOPROPYLTHIOUREE

### EXEMPLE 103 : N-[2-(3,4,5,6,7,8-HEXAHYDRO-2H-1-NAPHTO[2,1-b]PYRANN-5-YL) ETHYL] N'-METHYLUREE

### EXEMPLE 104 : N-[2-(2H-3,4,5,6,7,8-HEXAHYDRO-2H-1-NAPHTO[2,1-b]PYRANN-5-YL) ETHYL] N'-ETHYLUREE

### EXEMPLE 105 : N-[2-(3,4,5,6,7,8-HEXAHYDRO-2H-1-NAPHTO[2,1-b]PYRANN-5-YL) ETHYL] N'-PROPYLUREE

### EXEMPLE 106 : N-[2-(3,4,5,6,7,8-HEXAHYDRO-2H-1-NAPHTO[2,1-b]PYRANN-5-YL) ETHYL] N'-CYCLOPROPYLUREE

### EXEMPLE 107 : N-[2-(3,4,5,6,7,8-HEXAHYDRO-2H-1-NAPHTO[2,1-b]PYRANN-5-YL) ETHYL] N'-PROPYLTHIOUREE

### EXEMPLE 108 : N-[2-(3,4,5,6,7,8-HEXAHYDRO-2H-1-NAPHTO[2,1-b]PYRANN-5-YL) ETHYL] N'-CYCLOPROPYLTHIOUREE

### EXEMPLE 109 : N-[2-(3,4-DIHYDRO-2H-1-NAPHTO[2,1-b]PYRANN-5-YL)ETHYL] N'-METHYLUREE

### EXEMPLE 110 : N-[2-(3,4-DIHYDRO-2H-1-NAPHTO[2,1-b]PYRANN-5-YL)ETHYL] N'-ETHYLUREE

### EXEMPLE 111 : N-[2-(3,4-DIHYDRO-2H-1-NAPHTO[2,1-b]PYRANN-5-YL)ETHYL] N'-PROPYLUREE

### EXEMPLE 112 : N-[2-(3,4-DIHYDRO-2H-1-NAPHTO[2,1-b]PYRANN-5-YL)ETHYL] N'-CYCLOPROPYLUREE

### EXEMPLE 113 : N-[2-(3,4-DIHYDRO-2H-1-NAPHTO[2,1-b]PYRANN-5-YL)ETHYL] N'-PROPYLTHIOUREE

### EXEMPLE 114 : N-[2-(3,4-DIHYDRO-2H-1-NAPHTO[2,1-b]PYRANN-5-YL)ETHYL] N'-CYCLOPROPYLTHIOUREE

### EXEMPLE 115: N-[2-(7H-8,9-DIHYDROPYRANNO[3,2-e]INDOLYL)ETHYL]ACETAMIDE

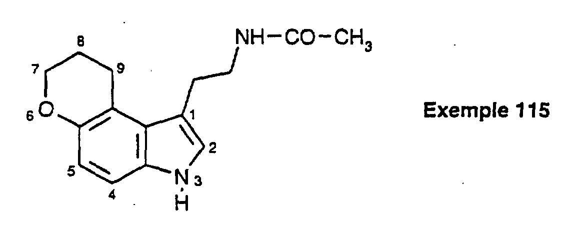

En procédant à une réaction d'acylation du composé de la préparation 39 avec le chlorure d'acétyle, on obtient le composé du titre.

### EXEMPLES 116 A 118 :

En procédant comme dans l'exemple 115, mais en utilisant le chlorure d'acyle approprié, on obtient les composés des exemples suivants :

### EXEMPLE 116: N-[2-(7H-8,9-DIHYDROPYRANNO[3,2-e]INDOLYL)ETHYL] PROPIONAMIDE

### EXEMPLE 117 : N-[2-(7H-8,9-DIHYDROPYRANNO[3,2-e]INDOLYL)ETHYL] CYCLOPROPANECARBOXAMIDE

### EXEMPLE 118: N-[2-(7H-8,9-DIHYDROPYRANNO[3,2-e]INDOLYL)ETHYL] CYCLOBUTYLCARBOXAMIDE

### EXEMPLE 119 : 2,3-DIHYDRO-2-METHYL-4-(2-ACETAMIDOETHYL)-1-NAPHTO[2,1-b] FURANNE

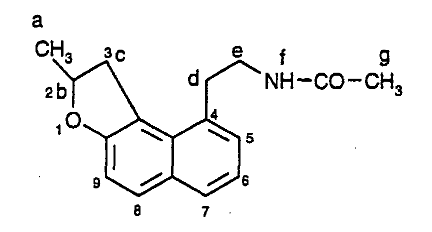

| **Réactifs :** | |
|---|---|
| N-[2-(8-allyl-7-hydroxy-napht-1-yl)éthyl]acétamide (Préparation 40) | 3,7 mmol (1 g) |
| acide trifluoroacétique (99 %, d = 1,48) | 32 mmol (2,33 cm³) |

### Mode opératoire :

Dans une fiole de 50 cm³, dissoudre le composé de la préparation 40 dans l'acide trifluoroacétique et porter le mélange à reflux pendant 8h. Laisser refroidir. Evaporer le milieu à sec. Reprendre par de l'eau et extraire à l'acétate d'éthyle (3 x 10 cm³). Laver la phase organique par 2 x 2 cm³ d'une solution aqueuse de soude à 10 % puis à l'eau. Sécher la phase organique sur MgSO₄ et la porter à sec. Purifier sur une colonne de silice en utilisant comme éluant l'Acétone-Toluène-Cyclohexane.

### Caractéristiques :

Masse moléculaire : 269,34 g pour C₁₇ H₁₉ NO₂
aspect : solide blanchâtre
Point de fusion : 136°C
Rf : 0,32 éluant : Acétone/Toluène/Cyclohexane (4/4/2)
Rendement : 73 %
Solvant de recristallisation : Toluène/Cyclohexane (1/3)

| **Infra-rouge :** | | |
|---|---|---|
| 3240 et 3050 | cm⁻¹ | ν NH amide |
| 2960-2840 | cm⁻¹ | ν CH alkyles |
| 1630 | cm⁻¹ | ν CO amide |
| 1000-1580 | cm⁻¹ | ν C=C aromatiques |

| **RMN (CDCl**_{**3**}**, δ) 300 MHz:** | | | | | |
|---|---|---|---|---|---|
| 1,54 | ppm | doublet | 3H | Ha | J_{a-b} = 6,30 Hz |
| 1,96 | ppm | singulet | 3H | Hg | |
| 3,29 | ppm | multiplet | 2H | Hd | |
| 3,40 | ppm | doublet de doublet | 1H | Hc' "cis" | J = 7,6 Hz ; J_{c'-b} = 7,7 Hz ; |
| | | | | | J_{c'-c} = 15,2 Hz |
| 3,56 | ppm | multiplet | 2H | He | |
| 3,94 | ppm | doublet de doublet | 1H | Hc "trans" | J_{c-b} = 9,2 Hz ; |
| | | | | | J_{c-c'} = 15,2 Hz |
| 5,05-5,07 | ppm | massif | 1H | Hb | |
| 5,53 | ppm | signal | 1H | Hf | |
| 7,08-7,23 | ppm | massif | 3H | H aromatiques : H5,6,9 | |
| 7,67-7,70 | ppm | massif | 2H | H aromatiques : H8,7 | |

### EXEMPLE 120 : N-[2-(2,3-DIHYDRO-2-METHYL-1-NAPHTO[2,1-b]FURANN-4-YL)ETHYL] N'-METHYLUREE

En procédant comme dans l'exemple 119 mais en utilisant au départ le composé de la préparation 41, on obtient le produit du titre.
Point de fusion : 165-169°C.

### EXEMPLES 121 A 130

### EXEMPLE 121 : N-[2-(7H-8,9-DIHYDRO-THIENO[3,2-f]BENZOPYRANN-1-YL)ETHYL] ACETAMIDE

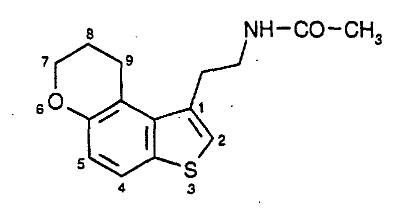

### EXEMPLE 122 : N-[2-(7H-8,9-DIHYDRO-THIENO[3,2-f]BENZOPYRANN-9 -YL)ETHYL] PROPIONAMIDE

### EXEMPLE 123 : N-[2-(7H-8,9-DIHYDRO-THIENO[3,2-f]BENZOPYRANN-9-YL)ETHYL] CYCLOPROPYLCARBOXAMIDE

### EXEMPLE 124 : N-[2-(7H-8,9-DIHYDRO-THIENO[3,2-f]BENZOPYRANN-9-YL)ETHYL] CYCLOBUTYLCARBOXAMIDE

### EXEMPLE 125 : N-[2-(7H-8,9-DIHYDRO-THIENO[3,2-f]BENZOPYRANN-9-YL)ETHYL] TRIFLUOROACETAMIDE

### EXEMPLE 126 : N-[2-(7H-8,9-DIHYDRO-FURO[3,2-f]BENZOPYRANN-1-YL)ETHYL] ACETAMIDE

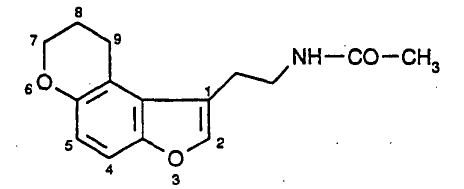

### EXEMPLE 127 : N-[2-(7H-8,9-DIHYDRO-FURO[3,2-f]BENZOPYRANN-1-YL)ETHYL] PROPIONAMIDE

### EXEMPLE 128 : N-[2-(7H-8,9-DIHYDRO-FURO[3,2-f]BENZOPYRANN-1-YL)ETHYL] CYCLOPROPYLCARBOXAMIDE

### EXEMPLE 129 : N-[2-(7H-8,9-DIHYDRO-FURO[3,2-f]BENZOPYRANN-1-YL)ETHYL] CYCLOBUTYLCARBOXAMIDE

### EXEMPLE 130 : N-[2-(7H-8,9-DIHYDRO-FURO[3,2-f]BENZOPYRANN-1-YL)ETHYL] TRIFLUOROACETAMIDE

### ETUDE PHARMACOLOGIQUE

### EXEMPLE A : ETUDE DE LA TOXICITE AIGUE

La toxicité aiguë a été appréciée après administration orale à des lots de 8 souris (26 ±2 grammes). Les animaux ont été observés à intervalles réguliers au cours de la première journée et quotidiennement pendant les deux semaines suivant le traitement. La DL₅₀, entraînant la mort de 50 % des animaux, a été évaluée.

La DL₅₀ des produits testés est supérieure à 1000 mg.kg⁻¹ pour les composés étudiés ce qui indique la faible toxicité des composés de l'invention.

### EXEMPLE B : ETUDE DE LIAISON AUX RECEPTEURS DE LA MELATONINE

### B1) ETUDE SUR DES CELLULES DE LA PARS TUBERALIS DE MOUTON

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1 pp 1-4, 1989).

### PROTOCOLE

1) Les membranes de pars Tuberalis de mouton sont préparées et utilisées comme tissu cible dans des expériences de saturation pour déterminer les capacités et affinités de liaison pour la 2-¹²⁵I-iodomélatonine.
2) Les membranes de Pars tuberalis de mouton sont utilisées comme tissu cible, avec les différents composés à tester, dans des expériences de liaison compétitive par rapport à la 2-iodo-mélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

II apparaît que les composés de l'invention possèdent une très grande affinité pour les récepteurs de la mélatonine. En particulier, le composé de l'exemple 119 présente une affinité extrêmement puissante pour les récepteurs à la mélatonine, avec une IC₅₀ de 6,9.10⁻¹⁵ M.

### B2) ETUDE SUR DES MEMBRANES DE CELLULES DU CERVEAU DE POULET (GALLUS DOMESTICUS)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 128, pages 475-482, 1991). La 2-[¹²⁵I] mélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2[¹²⁵I] mélatonine
- mélatonine
- composés de l'invention

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correspondent aux valeurs de l'affinité montrent que la liaison des composés testés est très puissante.

### EXEMPLE C : TEST DES QUATRE PLAQUES

Les produits de l'invention sont administrés par voie oesophagienne à des lots de dix souris. Un lot reçoit du sirop de gomme. 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.
Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.
Un logiciel permet:
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### RESULTATS

Il apparaît clairement que les composés de l'invention permettent d'agir de façon puissante sur le rythme circadien via le système mélatoninergique.

### EXEMPLE E : ACTIVITE ANTIARYTHMIQUE

### PROTOCOLE

### (Ref : LAWSON J.W. et al. J. Pharmacol. Expert. Therap. 160 : 22-31, 1968)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : ACTIVITE ANTI-AGREGANTE PLAQUETTAIRE

### PROTOCOLE

(Ref.: Bertele V. et al. Science. 220 : 517-519, 1983
Ibid, Eur. J. Pharmacol. 85 : 331-333, 1982)

Les composés de l'invention (100 µg/ml) sont testés pour leur capacité d'inhiber l'agrégation plaquettaire irréversible induite par l'arachidonate de sodium (50µg/ml) dans du plasma de lapin enrichi en plaquettes.

Une inhibition de plus de 50 % de l'agrégation maximum indique une activité significative pour les composés de l'invention.

Ce test *in vitro* montre que les composés de l'invention sont de bons candidats pour le traitement des maladies cardiovasculaires, notamment les thromboses

### EXEMPLE G : PROLONGATION DU TEMPS DE SAIGNEMENT

### PROTOCOLE

(Ref.: Djana E. et al. Thrombosis Research, 15 : 191-197, 1979)
Butler K.D. et al. Thromb. Haemostasis. 47 : 46-49, 1982)

Les composés à tester sont administrés par voie orale (100 mg/kg) à un groupe de 5 souris 1h avant le sectionnement standardisé du bout de chaque queue (0,5 mm).

Les souris sont immédiatement suspendues verticalement, les queues étant immergées de 2 cm dans un tube à essai contenant une solution saline isotonique à 37°C.

Le temps requis pour que le saignement cesse pendant une période de 15 secondes est alors déterminé.

Une prolongation de plus de 50 % du temps de saignement relative à un groupe d'animaux contrôle est considérée comme significative pour les composés de l'invention.

Ce test *in vivo* confirme l'intérêt des composés de l'invention pour le traitement des pathologies cardiovasculaires puisque les composés de l'invention prolongent le temps de saignement.

### EXEMPLE H : TEST D'HYPOXIE HYPOBARE

### PROTOCOLE

(Ref.: Gotti B., et Depoortere H., Circ. Cerebrale, Congrès de Circulation Cérébrale,
Toulouse, 105-107, 1979)

Les composés à tester sont administrés par voie intrapéritonéale (100 mg/kg) à un groupe de 3 souris 30 minutes avant d'être placés dans une chambre à la pression hypobare de 20 cm Hg.

La prolongation du temps de survie par rapport à un groupe d'animaux traités avec le véhicule de plus de 100 % en absence d'effet dépresseur du système nerveux central indique une activité cérébroprotective des composés de l'invention.

### EXEMPLE I : COMPOSITION PHARMACEUTIQUE : COMPRIMES

| 1000 comprimés dosés à 5 mg de 5-(2-acétamidoéthyl)-2H-1-naphto[2,1-b]pyranne | |
|---|---|
| 5-(2-acétamidoéthyl)-2H-1-naphto[2,1-b]pyranne | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I): 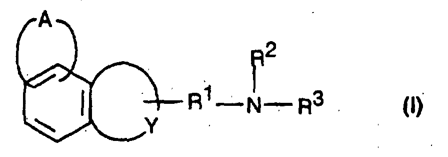 dans laquelle :
- R¹ représente une chaîne (C₁-C₄) alkylène non substituée ou substituée par un radical choisi parmi alkyle, hydroxy, alkoxycarbonyle et carboxyle ;
- R² représente un atome d'hydrogène ou un alkyle ;
- R³ représente :
. soit un groupement de formule R³¹ 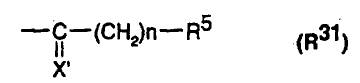 dans lequel n représente zéro ou un nombre entier de 1 à 3 et R⁵ représente un atome d'hydrogène, un alkyle non substitué ou substitué, un alcényle non substitué ou substitué, un alcynyle non substitué ou substitué, un cycloalkyle non substitué ou substitué, un dicycloalkylalkyle non substitué ou substitué ; et X' représente un atome d'oxygène ou de soufre ;
. soit un groupement de formule R³²: 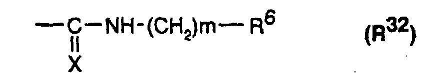 dans lequel X représente un atome d'oxygène ou de soufre,
m représente zéro ou un nombre entier de 1 à 3
et R⁶ représente un radical choisi parmi les même valeurs que R⁵ ;
- A représente une chaine de formule -O-A¹- dans laquelle A¹ est une chaine choisie parmi (C₂-C₅) alkylène, (C₂-C₅) alcénylène et (C₂-C₅) alcynylène ; A¹ étant non substitué ou substitué par un ou plusieurs groupements choisis parmi alkyle, alkoxy, hydroxy et oxo,
Y formant avec le noyau benzo auquel il est lié un groupement Y¹ choisi parmi naphtalène, naphtalène partiellement hydrogéné, benzofuranne, benzofuranne partiellement hydrogéné, benzothiophène, benzothiophène partiellement hydrogéné, et indole ;
étant entendu que :
- l'expression "substitué" affectant les termes "alkyle", "alcényle", et "alcynyle" signifie que ces groupements sont substitués par un ou plusieurs radicaux choisis parmi halogène, alkyle et alkoxy,
- l'expression "substitué" affectant le terme "cycloalkyle" ou "dicycloalkylalkyle" signifie que ces groupements sont substitués par un ou plusieurs radicaux choisis parmi : alkyle, alkoxy, hydroxy et le groupement oxo,
- les termes "alkyle" et "alkoxy" désignent des radicaux comportant de 1 à 6 atomes de carbone,
- les termes "alcényle" et "alcynyle" désignent des radicaux insaturés de 2 à 6 atomes de carbone,
- le terme "cycloalkyle" désigne un groupement de 3 à 8 atomes de carbone, saturé ou insaturé,
leurs énantiomères et diastéréoisomères,
et leurs sels d'addition à une base pharmaceutiquement acceptable.

2. Composé selon la revendication 1 qui est le 2,3-DIHYDRO-3-HYDROXY-4-(2-ACETAMIDOETHYL)-1-NAPHTO[2,1-b]FURANNE.

3. Composé selon la revendication 1 qui est le 2,3-DIHYDRO-2-METHYL-4-(2-ACETAMIDOETHYL)-1-NAPHTO[2,1-b]FURANNE.

4. Composé selon la revendication 1 qui est le 3,4-DIHYDRO-5-(2-ACETAMIDOETHYL)-2H-1-NAPHTO[2,1-b]PYRANNE.

5. Procédé de préparation des composés de formule (I) selon la revendication 1 **caractérisé en ce qu'**on cyclise un composé de formule (II) : 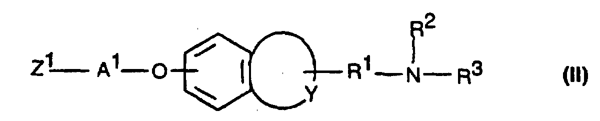 dans laquelle R¹, R², R³, A¹ et Y ont la même définition que dans la revendication 1 et Z¹ représente une fonction réactive telle qu'un groupement carboxyle, ester, chlorure d'acide, allyle ou alcyne,
afin d'obtenir le composé de formule (I) correspondant, 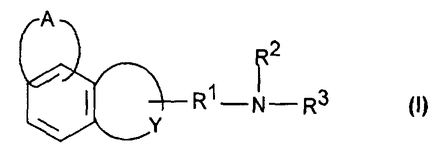 dans laquelle R¹, R², R³ et Y sont tels que définis précédemment et A est tel que défini dans la revendication 1,
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

6. Composés de formule (II) : 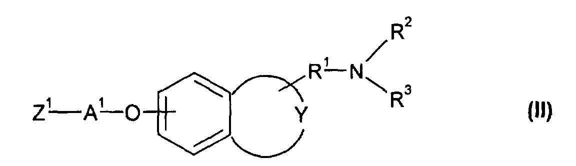 dans laquelle R¹, R², R³ et A¹ sont tels que définis dans la revendication 1 et Z¹ représente une fonction réactive
utiles en tant qu'intermédiaires de synthèse selon la revendication 5.

7. Procédé de préparation des composés de formule (I/d) cas particulier des composés de formule (I) : 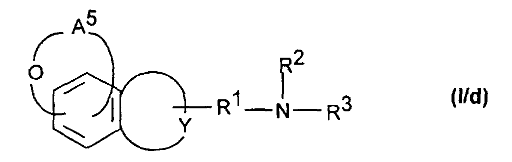 dans laquelle Y, R¹, R² et R³ sont tels que définis dans la revendication 1 et A⁵ représente une chaîne (C₂-C₅) alkylène non substituée ou substituée par un radical (C₁-C₆) alkyle **caractérisé en ce que** un composé de formule (VI): 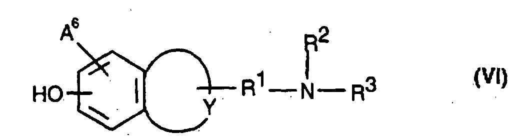 dans laquelle Y, R¹, R² et R³ sont tels que définis précédemment et A⁶ représente un radical (C₂-C₅) alcényle non substitué ou substitué par un radical (C₁-C₆) alkyle est soumis à une réaction de cyclisation,
les composés de formule (I/d) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

8. Composés de formule (VI) : 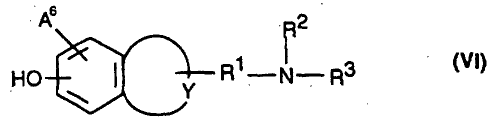 dans laquelle, R¹, R², R³ et Y sont tels que définis dans la revendication 1 et A⁶ représente un radical(C₂-C₅) alcényle non substitué ou substitué par un radical (C₁-C₆) alkyle, utiles comme intermédiaires de synthèse selon la revendication 7.

9. Compositions pharmaceutiques contenant les produits de formule (I) selon la revendication 1 ou le cas échéant un de leurs sels d'addition à une base pharmaceutiquement acceptable en combinaison avec un ou plusieurs excipients pharmaceutiquement acceptables.

10. Compositions selon la revendication 9 utiles dans le traitement des troubles du système mélatoninergique.

## Patentansprüche

1. Verbindungen der Formel (I): 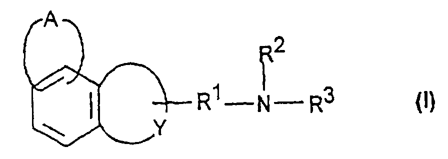 in der:
- R¹ (C₁-C₄)-Alkylenkette darstellt, die nicht substituiert oder durch einen Rest ausgewählt aus Alkyl, Hydroxy, Alkoxycarbonyl und Carboxyl substituiert ist;
- R² ein Wasserstoffatom oder eine Alkylgruppe bedeutet;
- R³:
. entweder eine Gruppe der Formel R³¹ 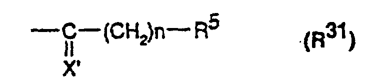 in der n Null oder eine ganze Zahl mit einem Wert von 1 bis 3 und R⁵ ein Wasserstoffatom, eine gegebenenfalls substituierte Alkylgruppe, eine gegebenenfalls substituierte Alkenylgruppe, eine gegebenenfalls substituierte Alkinylgruppe, eine gegebenenfalls substituierte Cycloalkylgruppe, eine gegebenenfalls substituierte Dicycloalkylalkylgruppe; und X' ein Sauerstoffatom oder ein Schwefelatom darstellen;
. oder eine Gruppe der Formel R³²: 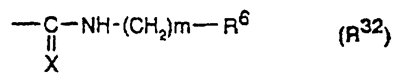 in der X ein Sauerstoffatom oder ein Schwefelatom,
m Null oder eine ganze Zahl mit einem Wert von 1 bis 3 und
R⁶ eine Gruppe ausgewählt aus den für R⁵ angegebenen Gruppen darstellen, bedeutet;
- A eine Kette der Formel -O-A¹- darstellt, worin A¹ eine Kette ausgewählt aus ((C₂-C₅)-Alkylen, (C₂-C₅)-Alkenylen und (C₂-C₅)-Alkinylen darstellt, worin A¹ unsubstituiert ist oder durch eine oder mehrere Gruppen ausgewählt aus Alkyl, Alkoxy, Hydroxy und Oxo substituiert ist,
- Y mit dem Benzokern, an den es gebunden ist, eine Gruppe Y¹ bildet, welche aus Naphthalin, teilweise hydriertem Naphthaltin, Benzofuran, teilweise hydriertem Benzofuran, Benzothiophen, teilweise hydriertem Benzothiophen und Indol ausgewählt ist;
wobei es sich versteht, daß:
- der Ausdruck "substituiert", wie er für die Begriffe "Alkyl", "Alkenyl" und "Alkinyl" benutzt wird, bedeutet, daß diese Gruppen durch einen oder mehrere Reste ausgewählt aus Halogen, Alkyl und Alkoxy substituiert sind,
- der Ausdruck "substituiert", wie er für den Begriff "Cycloalkyl" oder "Dicycloalkylalkyl" benutzt wird, bedeutet, daß diese Gruppen durch einen oder mehrere Reste ausgewählt aus Alkyl, Alkoxy, Hydroxy und die Oxogruppe substituiert sind,
- die Begriffe "Alkyl" und "Alkoxy" für Reste stehen, die 1 bis 6 Kohlenstoffatome umfassen,
- die Begriffe "Alkenyl" und "Alkinyl" für ungesättigte Reste mit 2 bis 6 Kohlenstoffatomen stehen und
- der Begriff "Cycloalkyl" für ene gesättigtge oder ungesättigte Gruppe mit 3 bis 8 Kohlenstoffatomen steht,
deren Enantiomere und Diastereoisomere sowie deren Additionssalze mit einer pharmazeutisch annehmbaren Base.

2. Verbindung nach Anspruch 1, nämlich 2,3-Dihydro-3-hydroxy-4-(2-acetamidoethyl)-1-naphtho[2,1-b]furan.

3. Verbindung nach Anspruch 1, nämlich 2,3-Dihydro-2-mehyl-4-(2-acetamidoethyl)-1-naphtho[2,1-b]furan.

4. Verbindung nach Anspruch 1, nämlich 3,4-Dihydro-5-(2-acetamidoethyl)-2H-1-naphtho[2,1-b]pyran.

5. Verfahren zur Herstellung der Verbindungen der Formel (I) nach Anspruch 1, **dadurch gekennzeichnet, daß** man eine Verbindung der Formel (II): 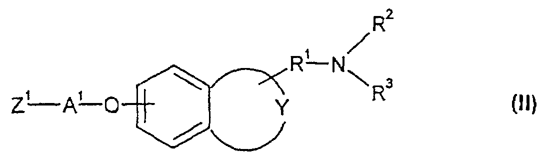 in der R¹, R², R³, A¹ und Y die in Anspruch 1 angegebenen Bedeutungen besitzen und Z¹ eine reaktive Gruppe, wie eine Carboxylgruppe, eine Estergruppe, eine Säurechloridgruppe, eine Allylgruppe oder eine Alkingruppe darstellt, cyclisiert
zur Bildung der entsprechenden Verbindung der Formel (I) 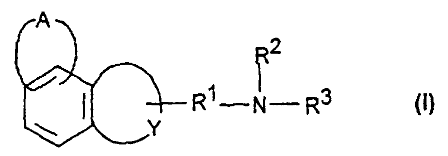 in der in der R¹, R², R³ und Y die oben angegebenen Bedeutungen besitzen und A die in Anspruch 1 angegebenen Bedeutungen besitzt,
welche Verbindungen der Formel (I) gewünschtenfalls
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können,
- oder mit einer pharmazeutisch annehmbaren Base in die Salze überführt werden können.

6. Verbindungen der Formel (II): 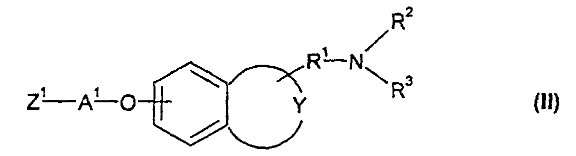 in der R¹, R², R³ und A¹ die in Anspruch 1 angegebenen Bedeutungen besitzen und Z¹ eine reaktive Gruppe darstellt,
als Zwischenprodukte für die Synthese gemäß Anspruch 5.

7. Verfahren zur Herstellung der Verbindungen der Formel (I/d), einem Sonderfall der Verbindungen der Formel (I): 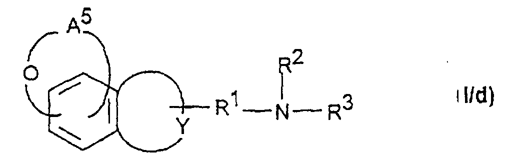 in der Y, R¹, R² und R³ die in Anspruch 1 angegebenen Bedeutungen besitzer und A⁵ eine (C₂-C₅)-Alkylenkette darstellt, die nicht substituiert oder durch einen (C₁-C₆)-Alkylrest substituiert ist,
**dadurch gekennzeichnet, daß** man eine Verbindung der Formel (VI): 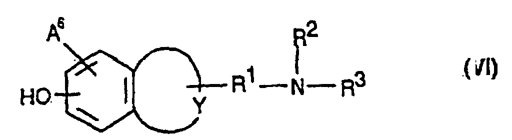 in der Y, R¹, R² und R³ die oben angegebenen Bedeutungen besitzen und A⁶ einen (C₂-C₅)-Alkenylrest darstellt, der nicht substituiert oder durch einen in der (C₁-C₆)-Alkylrest substituiert ist, einer Cyclisierungsreaktion urterwirft,
wobei man die Verbindungen der Formel (I/d) gewünschtenfalls
- mit Hilfe einer oder mehrerer Reinigungsmethoden ausgewählt aus Kristallisation, Chromatographie über Kieselgel, Extraktion, Filtration und Überführung über Aktivkohle oder ein Harz reinigen kann,
- gegebenenfalls in ihre eventuellen Enantiomeren oder Diastereoisomeren in reiner Form oder in Form einer Mischung trennen kann,
- oder mit einer pharmazeutisch annehmbaren Base in ihre Salze überführen kann.

8. Verbindungen der Formel (VI): 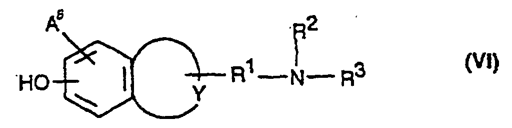 in der R¹, R², R³ und Y die in Anspruch 1 angegebenen Bedeutungen besitzen und A⁶ einen (C₂-C₅)-Alkenylrest darstellt, der nicht substituiert oder durch einen (C₁-C₆)-Alkylrest substituiert ist, als Zwischenprodukte für die Synthese nach Anspruch 7.

9. Pharmazeutische Zubereitungen enthaltend die Produkte der Formel (I) nach Anspruch 1 oder gegebenenfalls eines ihrer Additionssalze mit einer pharmazeutisch annehmbaren Base in Kombination mit einem oder mehreren pharmazeutisch annehmbaren Trägermaterialien.

10. Zubereitungen nach Anspruch 9 zur Behandlung von Störungen des melatoninergischen Systems.

## Claims

1. Compounds of formula (I): 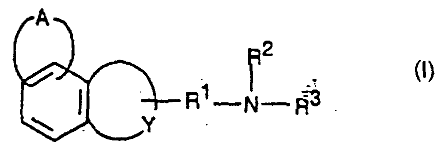 in which:
- R¹ represents a (C₁-C₄)alkylene chain that is unsubstituted or is substituted by a radical selected from alkyl, hydroxy, alkoxycarbonyl and carboxyl;
- R² represents a hydrogen atom or an alkyl radical;
- R³ represents:
. either a group of formula R³¹ 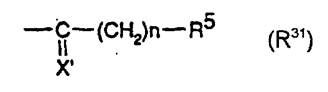 in which n represents zero or an integer from 1 to 3 and R⁵ represents a hydrogen atom, an unsubstituted or substituted alkyl radical, an unsubstituted or substituted alkenyl radical, an unsubstituted or substituted alkynyl radical, an unsubstituted or substituted cycloalkyl radical, an unsubstituted or substituted dicycloalkylalkyl radical; and X' represents an oxygen or sulphur atom;
. or a group of formula R³² 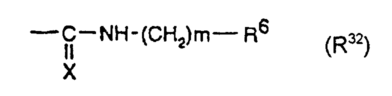 in which X represents an oxygen or sulphur atom,
m represents zero or an integer from 1 to 3,
and R⁶ represents a radical selected from the same meanings as R⁵;
- A represents a chain of the formula -O-A¹ in which A¹ is a chain selected from (C₂-C₅)alkylene, (C₂-C₅)alkenylene and (C₂-C₅)alkynylene; A¹ being unsubstituted or substituted by one or more groups selected from alkyl, alkoxy, hydroxy and oxo,
Y forming with the benzene ring to which it is attached a group Y¹ selected from naphthalene, partially hydrogenated naphthalene, benzofuran, partially hydrogenated benzofuran, benzothiophene, partially hydrogenated benzothiophene and indole;
wherein:
- the expression "substituted" associated with the terms "alkyl", "alkenyl" and "alkynyl" means that those groups are substituted by one or more radicals selected from halogen, alkyl and alkoxy,
- the expression "substituted" associated with the term "cycloalkyl" or "dicycloalkylalkyl" means that those groups are substituted by one or more radicals selected from: alkyl, alkoxy, hydroxy and the oxo group,
- the terms "alkyl" and "alkoxy" denote radicals having from 1 to 6 carbon atoms,
- the terms "alkenyl" and "alkynyl" denote unsaturated radicals having from 2 to 6 carbon atoms,
- the term "cycloalkyl" denotes a group, saturated or unsaturated, having from 3 to 8 carbon atoms,
their enantiomers and diastereoisomers,
and addition salts thereof with a pharmaceutically acceptable base.

2. Compound according to claim 1 which is 2,3-DIHYDRO-3-HYDROXY-4-(2-ACETAMIDOETHYL)-1-NAPHTHO[2,1-b]FURAN.

3. Compound according to claim 1 which is 2,3-DIHYDRO-2-METHYL-2-(2-ACETAMIDOETHYL)-1-NAPHTHO[2,1-b]FURAN.

4. Compound according to claim 1 which is 3,4-DIHYDRO-5-(2-ACETAMIDOETHYL)-2H-1-NAPHTHO[2,1-b]PYRAN.

5. Process for the preparation of compounds of formula (I) according to claim 1, which process is **characterised in that** a compound of formula (II): 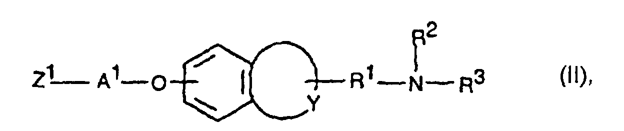 in which R¹, R², R³, A¹ and Y are as defined in claim 1 and Z¹ represents a reactive function such as a carboxyl, ester, acid chloride, allyl, or alkyne group, is cyclised in order to obtain the corresponding compound of formula (I) 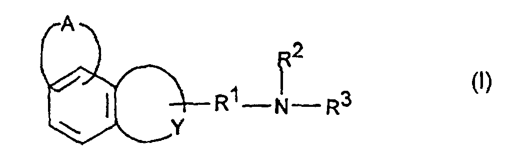 in which R¹, R², R³ and Y are as defined above and A is as defined in claim 1,
which compounds of formula (I) may, if desired, be
- purified by one or more purification methods selected from crystallisation, chromatography on silica gel, extraction, filtration, and passage over charcoal or resin,
- separated, where appropriate, in pure form or in the form of a mixture, into their possible enantiomers or diastereoisomers,
- or converted into salts with a pharmaceutically acceptable base.

6. Compounds of formula (II): 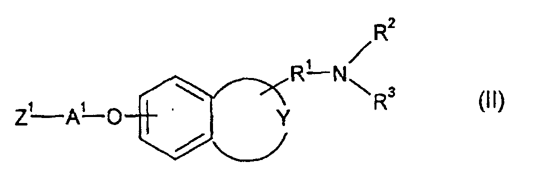 in which R¹, R², R³ and A¹ are as defined in claim 1 and Z¹ represents a reactive function,
for use as synthesis intermediates according to claim 5.

7. Process for the preparation of compounds of formula (I/d), a particular case of the compounds of formula (I) 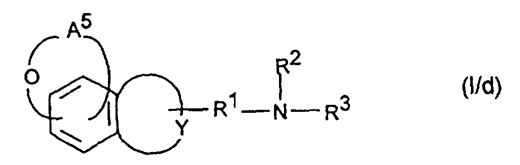 in which Y, R¹, R² and R³ are as defined in claim 1 and A⁵ represents a (C₂-C₅)alkylene chain that is unsubstituted or is substituted by a (C₁-C₆)alkyl radical,
which process is **characterised in that** a compound of formula (VI): 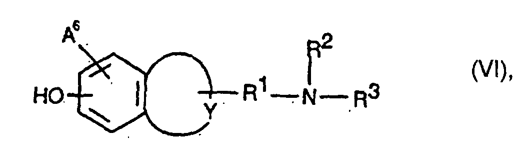 in which Y, R¹, R² and R³ are as defined above and A⁶ represents a (C₂-C₅)alkenyl radical that is unsubstituted or is substituted by a (C₁-C₆)alkyl radical,
is subjected to a cyclisation reaction,
which compounds of formula (I/d) may, if desired, be
- purified by one or more purification methods selected from crystallisation, chromatography on silica gel, extraction, filtration, and passage over charcoal or resin,
- separated, where appropriate, in pure form or in the form of a mixture, into their possible enantiomers or diastereoisomers,
- or converted into salts with a pharmaceutically acceptable base.

8. Compounds of formula (VI): 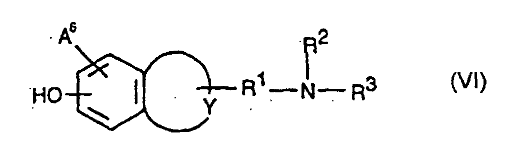 in which R¹, R², R³ and Y are as defined in claim 1 and A⁶ represents a (C₂-C₅)alkenyl radical that is unsubstituted or is substituted by a (C₁-C₆)alkyl radical,
for use as synthesis intermediates according to claim 7.

9. Pharmaceutical compositions comprising the compounds of formula (I) according to claim 1 or, where appropriate, an addition salt thereof with a pharmaceutically acceptable base, in combination with one or more pharmaceutically acceptable excipients.

10. Compositions according to claim 9 for use in the treatment of disorders of the melatoninergic system.
